# EUROPEAN PATENT APPLICATION

(11) **EP 4 079 846 A1**
(43) Date of publication of application: **26.10.2022**
(21) Application number: 21170068.7
(22) Date of filing: 23.04.2021
(51) Int. Cl.: C12N 9/02, C12Q 1/66, G01N 33/50

(54) **NOVEL LUCIFERASES WITH IMPROVED PROPERTIES**

(71) Applicant: SVAR Life Science AB, 202 11 Malmö (SE)
(72) Inventor: TOVEY, Michael, 75005 PARIS (FR); HANG, Lue, 75014 PARIS (FR); LALLEMAND, Christophe, 75018 PARIS (FR)
(74) Representative: Zacco Denmark A/S

(57) **Abstract**

The present invention relates to novel luciferase genes that exhibit improved characteristics when expressed in cells as a reporter gene and method of using the same for detecting and optionally quantitating the activity of pharmacologically active molecules in a test sample. The invention further relates to a method for detecting and optionally quantitating neutralizing antibodies against a pharmacologically active molecule present in a test sample using the reporter cell line of the present invention. The invention further relates to a method for high-throughput screening in drug discovery for detecting and optimally monitoring biologic processes with optimal sensitivity, signal strength, and biological fidelity using said luciferase genes as reporter genes.

## Description

### Field of the invention

The present invention relates to luciferase genes derived from the naturally occurring marine copepod *Metridia longa* luciferase gene and reporter-gene cell lines incorporating said luciferases as reporter genes and method of using the same for detecting and optimally quantitating gene expression in a test sample. The invention further relates to a method for detecting and optimally quantitating the activity of pharmacology active molecules present in a test sample and the neutralizing antibody response against said molecules using the reporter cell lines of the present invention. The invention further relates to a non-toxic method for high-throughput screening in drug discovery that permits multiple sampling for detecting and optimally monitoring biologic processes with optimal sensitivity, signal strength, and biological fidelity. A novel vector is also disclosed that contains both bacterial and mammalian selection markers, a multiple cloning site, transposon-specific inverted terminal repeats, an efficient minimal promoter and is ideally suited for the expression of a wide range of naturally occurring or engineered reporter-genes including but not limited to fluorescent proteins such as green fluorescent protein (GFP) or red fluorescent protein (RFP), or enzymes such as luciferase including but not limited to firefly luciferase, Renilla luciferase, Gaussia luciferase, or the novel luciferase derived from *Metridia longa* disclosed in the present invention.

### Background of the invention

Bioluminescent and fluorescent reporter-genes are used extensively for the study of gene expression and have found wide application in high-throughput screening (HTS) in drug discovery (Inglese et al, 2007) and for the quantification of drug activity (Lallemand et al. 2010, 2011, 2017). One of the principal advantages of luminescence is that in contrast to fluorescence it does not require excitation by an external light source resulting in a high signal to background ratio. Bioluminescence assays employ luciferase enzymes that catalyze the oxidation of luciferin substrates into oxyluciferin with the emission of light that can be quantified using a luminometer. Naturally occurring luciferases can be divided into two principal groups based on the use of D-luciferin/ATP or coelenterazine as a substrate (Thorne et al 2010). Naturally occurring luciferases may be expressed intracellularly as in the case of firefly luciferase (FL) or secreted such as Metridia luciferase (Markova et al 2004). Luciferases such as FL or Renilla luciferase have relatively short half-lives relative to fluorescent proteins such as GFP (Corish and Tyler-Smith 1999) and can be used to quantify dynamic changes in reporter-gene transcription levels allowing the quantification of the activity of intracellular gene expression or diverse extracellular signals to be quantified as we and others have shown (Lallemand et al. 2008, 2010, 2011, 2017). Luciferases can be combined in dual reporter-gene assays allowing the activity of an extracellular signal such as that following the interaction of a drug with a cell surface receptor to be quantified using for example FL under the control of a drug-responsive promoter and results can be normalized relative to the expression of a second luciferase such as Renilla luciferase (RL) under the control of a constitutive promoter either in transient transfection experiments or following stable transfection of cells with both reporter-gene constructs (Lallemand et al. 2010, 2011, 2017). This allows drug activity to be quantified using for example FL and results to be normalized for effects such as cytotoxicity or differences in cell number following quenching of FL expression and quantification of RL levels in the same well of a microtiter plate using a commercially available substrate (Lallemand et al. 2010, 2011, 2017).

Although naturally occurring luciferases have found wide application as reporter-genes for the study of gene expression and in high-throughput screening in drug discovery there is a need for improved luciferases that ideally should be extremely bright such that the product of single copy genes could be readily detected, exhibit a large dynamic range, secreted to give low background levels and allow serial sampling, use a "glo" substrate that does not require the use of a luminometer equipped with injectors, small in size to facilitate expression in transfected cells and to be non-toxic for cells when expressed at high levels.

It follows from the above that there is a need for an engineered luciferase that exhibits optimal characteristics for use as a reporter gene for the quantification of the activity of pharmacology active molecules and for high throughput screening in drug discovery.

There is also a need for a versatile dual bacterial-mammalian expression with both a conventional multiple cloning site and transposon-specific integration site, and an improved minimal promoter

### Summary of the invention

The present invention was made in view of the prior art described above, and the object of the present invention is to provide a novel luciferase gene optimized for use as a reporter-gene for the quantification of the activity of pharmacology active molecules and for use in high throughput screening in drug discovery.

In particular, the object of the invention is to provide for an optimized luciferase gene that encodes a luciferase protein that exhibits superior properties in comparison to that encoded by the native gene comprising, but not limited to, one or more of;
(i): increased light output (brightness),
(ii): enhanced signal stability and/or signal duration as reflected by high Relative Light Units (RLU),
(iii): exhibits a large dynamic range relative to control samples in the absence of the pharmacology active substance,
(iv): is secreted to give low background levels and to allow serial sampling,
(v): has a sufficiently long half-life to obviate the need for the use of a luminometer equipped with injectors,
(vi): is small in molecular size and codon-optimized to facilitate expression in heterologous transfected cells,
(vii): exhibits enhanced biocompatibility comprising at least one of the following characteristics; of improved expression in cells and reduced toxicity when expressed at high levels.

The elements of the invention provides for one or more of the above mentioned effects.

In various embodiments, the present invention comprises novel luciferases that are present in solution as soluble active monomers, or as fusion proteins with other proteins including luciferases or fluorescent proteins such as green fluorescent proteins (GFPs) enhanced green fluorescent protein (EGFP), red fluorescent proteins (RFPs), yellow fluorescent protein (YFP), blue fluorescent protein (BFP) and variant there of displaying a different excitation/emission spectra, or various linker proteins, or attached to solid surfaces such as particles, assay-plates or tubes.

To solve the problem, the present invention provides a novel luciferase gene comprising, in one embodiment of the present invention at least one of (i)-(iii):
(i) An open reading frame encoding a luciferase protein, that has been codon-optimized for expression in said cell.

In a particular embodiment the open reading frame may comprise or consist of the nucleotide sequence set forth in SEQ ID NO: 1 and encoding the amino acid sequence set forth in SEQ ID NO: AA1.

In a further embodiment, the open reading frame may comprise or consist of the nucleotide sequence set forth in SEQ ID NO: 2 and encoding the amino acid sequence set forth in SEQ ID NO: AA2.
(ii) a signal peptide, whether from a homologous or heterologous species, to ensure efficient secretion from the cell.

In one aspect, the open reading frame comprises or consist of the sequence set forth in SEQ ID NO: 3 enabling efficient secretion from the cell.
(iii) a novel bacterial expression vector containing a minimal promoter from the 5' UTR of the human interferon beta gene, the codon-optimized signal peptide of the Gaussia luciferase gene, and 5' and 3' transposable repeats recognized by transposases for the insertion of a transgene or reporter-gene, which may comprise, the sequences set forth in SEQ ID NO: 1 and SEQ ID NO: 2, and the Nourseothricin antibiotic resistance gene or other antibiotic resistance gene such as e.g. ampicillin or kanamycin under the control of a EM7 minimal promoter or a composite CMV T7 promoter that is functional in both bacterial and mammalian cells, and the codon optimized hygromycin resistance gene, that comprises or consist of the sequence set forth in SEQ ID NO:4, or other mammalian antibiotic resistance gene such as neomycin, puromycin, zeocin, or blasticidin.

In one aspect, the bacterial expression vector may be a plasmid which in its entirety may comprise or consist of the sequence set forth in SEQ ID NO: 5

Thus, in one aspect, SEQ ID NO.: 5 comprises or consist of SEQ ID NO :1, SEQ ID NO:3, and SEQ ID NO4.

Another aspect of the present invention provides a method for detecting and optionally quantitating the activity of a pharmacology active molecule in a test sample, said method comprising the steps of
(i) providing a test sample,
(ii) contacting said test sample with the cell line according to the present invention, said cell line containing a first heterologous polynucleotide comprising a heterologous cis-acting regulatory sequence, that responds to treatment of the cell line with the pharmacology active molecule(s) present in the test sample, operably linked to a downstream promoter sequence, wherein said promoter is operably linked to an open reading frame encoding a first reporter protein comprising one of the sequences set forth in SEQ ID NO: AA1 and SEQ ID NO: AA2
(iii) In a preferred embodiment, to provide for a normalization of the assay, the cell lines according to the present invention further have a construct for the constitutive production of a luciferase that is different from that used in the reporter gene construct that responds to a pharmacology active molecule. For example, the constitutive production may be of a second luciferase, e.g., Renilla luciferase or firefly luciferase.
(iv) determining the activity of the first reporter protein in said cell line using a colenterazine based substrate such as that the composition of which is shown in Table 1 or a commercially available substrate such as Quanti-Luc Gold (InvivoGen), or any suitable commercially available coelenterazine based substrate,
(v) determining the activity of the second reporter protein in said cell line after the reporter gene luciferase is measured in the same sample using the Stop & Glo reagent from the Dual-Glo system (Promega) that efficiently inhibits the activity of the first reporter protein comprising one the sequences set forth in SEQ ID NO: 1 and SEQ ID NO: 2 in the absence of colenterazine.
(vi) The activity of the first luciferase normalized relative to the activity of the second luciferase is described in US 2011/0189658 incorporated herein by reference in its entirety.
(vii) In a further preferred embodiment, to provide for a dual-luciferase assay using different substrates, the cells according to the present invention further have a dual or poly-cistronic construct operably linked to an open reading frame encoding a luciferase reporter protein, wherein said open reading frame comprises or consist of the nucleotide sequence set forth in SEQ ID NO: 1 or SEQ ID NO: 2 and a second open reading frame encoding firefly luciferase or any other luciferase that uses luciferin as a substrate separated by the coding sequence of a self-cleaving 2A peptide F2A set forth in SEQ ID NO: 6
(viii) determining the activity of the first reporter protein in said cell line using a coelenterazine based substrate such as that the composition of which is shown in Table 1 or a commercially available substrate such as Quanti-Luc Gold (InvivoGen), or any suitable commercially available coelenterazine based substrate,
(ix) determining the activity of the second reporter protein in said cell line after the reporter gene luciferase is measured in the same sample using the BrightGlo reagent (Promega), or another commercially available luciferin based substrate,
(x) In a further preferred embodiment, to provide for a tri-luciferase assay using different substrates, the cells according to the present invention further have 3 constructs each operably linked to different ligand-responsive promoter operationally linked an open reading frame encoding a luciferase reporter protein, wherein said open reading frame comprises or consist of the nucleotide sequence set forth in SEQ ID NO: 1 or SEQ ID NO: 2 and a second open reading frame encoding firefly luciferase or any other luciferase that uses luciferin as a substrate together with third open reading frame encoding Renilla luciferase or any other luciferase that uses coelenterazine as a substrate. Alternatively, Renilla luciferase can be operationally linked to a constitutive promoter to allow the activity of the two ligand responsive luciferase to be normalized relative to the activity of a luciferase under the control of a constitutive promoter described in US 2011/0189658 which is incorporated herein by reference in its entirety.
(xi) determining the activity of the first reporter protein wherein said open reading frame comprises or consist of the nucleotide sequence set forth in SEQ ID NO: 1 or SEQ ID NO: 2 under the control of a signal peptide that ensures efficient secretion of the first reporter protein into the culture medium or supernatant of said cell line using a coelenterazine based substrate such as that the composition of which is shown in Table 1 or a commercially available substrate such as Quanti-Luc Gold (InvivoGen), or another commercially available coelenterazine based substrate,
(xii) determining the activity of the second reporter protein requiring a luciferin based substrate such as firefly luciferase in said cell line following cell lysis using the DualGlo reagent (Promega) after which the activity of the third reporter gene such as Renilla luciferase that requires a coeleranterize based substrate is measured in the same sample using the DualGlo reagent (Promega), or using a commercially available two component substrate the first component of which is a luciferin-based substrate that allows the substrate allowing the quantification of firefly luciferase activity and a second component that quenches firefly luciferase activity and contains a coelenterazine based substrate allowing quantification of Renilla luciferase activity.

A further aspect of the present invention concerns a method for high throughput screening in drug discovery said method comprising the steps of:
(i) providing a test sample(s), consisting of a library of the compounds to be screened,
(ii) contacting said test sample(s) in (i) with the cell line according to the present invention, said cell line containing a first heterologous polynucleotide comprising a heterologous cis-acting regulatory sequence, that responds to treatment of the cell line with the pharmacology active molecule(s) present in the test sample(s), operably linked to a downstream promoter sequence, wherein said promoter is operably linked to an open reading frame encoding a first reporter protein comprising one the sequences set forth in SEQ ID NO: 1 and SEQ ID NO: 2. In a preferred embodiment the cell line according to the present invention, is seeded in well plates or assay plates.
(iii) Treatment of the cell line according to the present invention with the agent(s) used to screen the library.
(iv) In a preferred embodiment, to provide for a normalization of the assay, the cell lines according to the present invention further have a construct for the constitutive production of a luciferase that is different from that used in the reporter gene construct. For example, the constitutive production may be of a second luciferase, e.g., Renilla luciferase or firefly luciferase.
(v) determining the activity of the first reporter protein in said cell line using a coelenterazine based substrate such as that the composition of which is shown in Table 1 or a commercially available substrate such as Quanti-Luc Gold (InvivoGen), or another commercially available coelenterazine based substrate,
(vi) determining the activity of the second reporter protein in said cell line after the reporter gene luciferase is measured in the same sample using Bright Glo (Promega), or a commercially available luciferin based substrate that efficiently inhibits the activity of the first reporter protein comprising one the sequences set forth in SEQ ID NO: AA1 and SEQ ID NO: AA2 in the absence of coelenterazine.
(vii) The activity of the first luciferase normalized relative to the activity of the second luciferase is described in US 2011/0189658 incorporated herein by reference in its entirety.

A further aspect of the present invention concerns a method detecting and optionally quantitating neutralizing antibodies against a pharmacology active molecule(s) present in the test sample, said method comprising the steps of
(i) providing a test sample comprising a pharmacology active molecule(s),
(ii) providing a first and a second cell sample, wherein said cells samples comprises the cell line according to the present invention,
(iii) contacting the first cell sample with the test sample and subsequently with the pharmacology active molecule,
(iv) contacting the second cell sample with the pharmacology active molecule,
(iv) determining the activity of the first reporter protein in the cells of the first cell sample and determining the activity of the first reporter protein in the cells of the second cell sample,
(v) provide the ratio between the reporter activity in first and a second cell sample, where a ratio (first/second) lower than one is indicative for the presence of antibodies against the pharmacology active molecule in said sample.

Consequently, the present invention provides for luciferase genes and cell lines transfected with said genes, the use thereof in various contexts of the invention allowing for the detection of the activity of a pharmacology active molecule with a higher sensitivity, such that the EC₅₀ of cells transfected according to the invention is at least decreased in comparison with cells transfected with the corresponding native gene or genes. The gene may be e.g. the native *Metridia longa* luciferase gene but may otherwise comprise other and/or further genes. The decrease may be about 2-fold, such as e.g. about 3-fold, such as e.g. about 4-fold, such as e.g. about 5-fold, such as e.g. 6-fold, such as e.g. 7-fold, such as e.g. about 8-fold, such as e.g. 9-fold, such as e.g. 10-fold, such as e.g. 50-fold, such as about 100-fold, or such as about 1000-fold.

### Brief description of the drawings

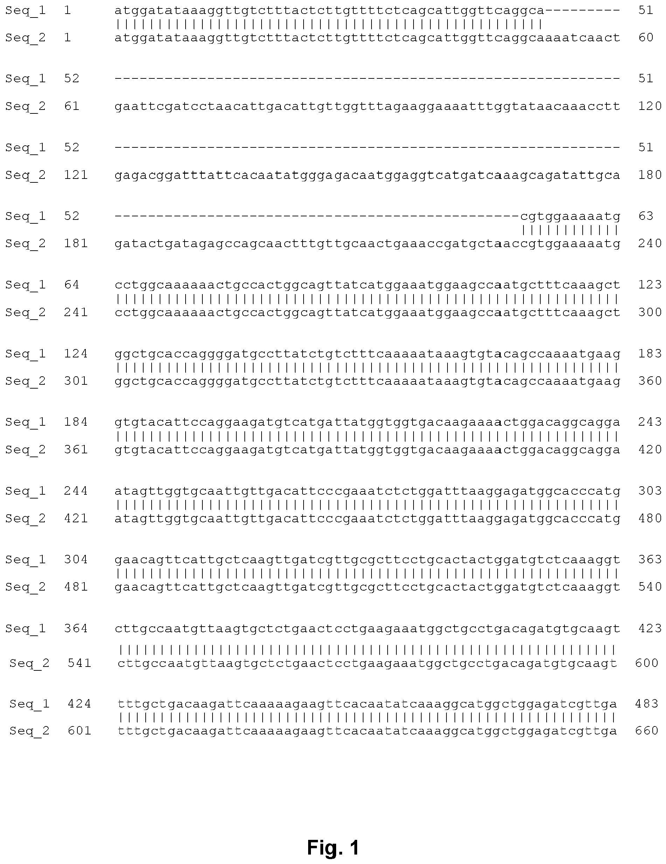

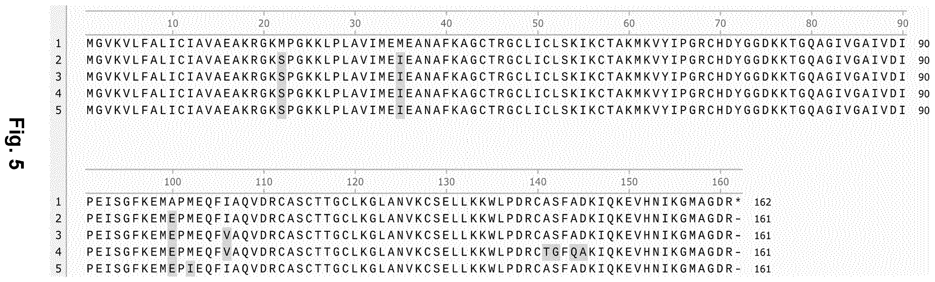

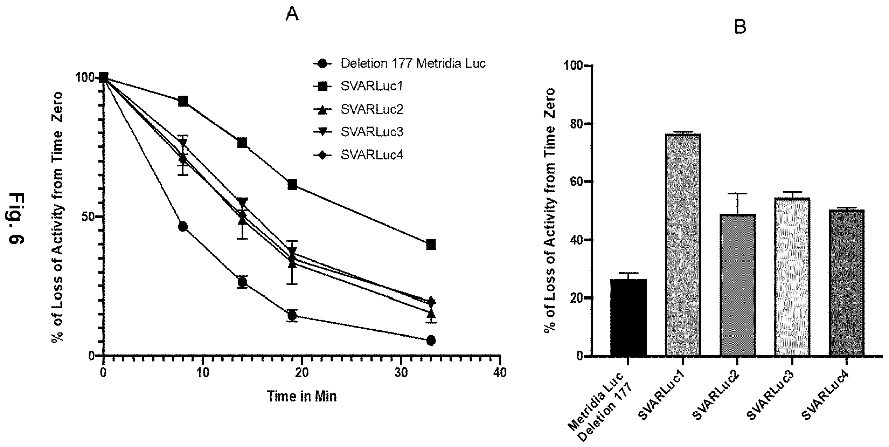

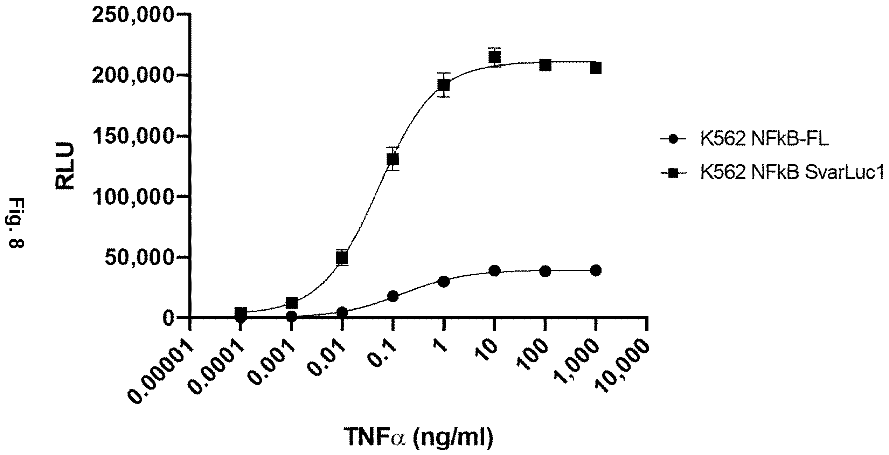

Fig. 1 illustrates the nucleotide sequence alignment of the wild type *Metridia longa* luciferase gene and a deletion mutant (nt. 51 to nt. 228) of the 5' translated region of the gene.

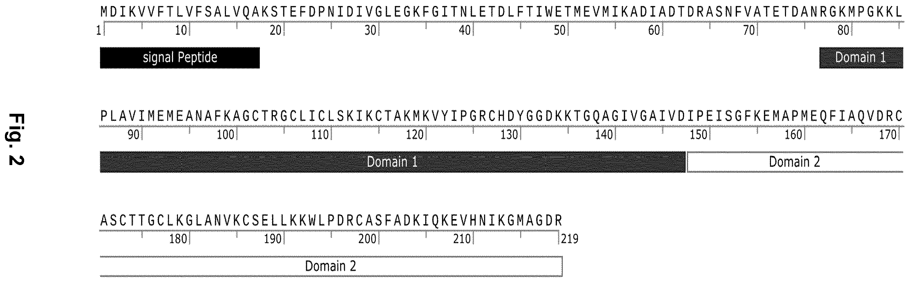

Fig. 2 illustrates the amino-acid sequence of the native *Metridia longa* luciferase showing the position of the signal peptide and the putative catalytic domains 1 and 2

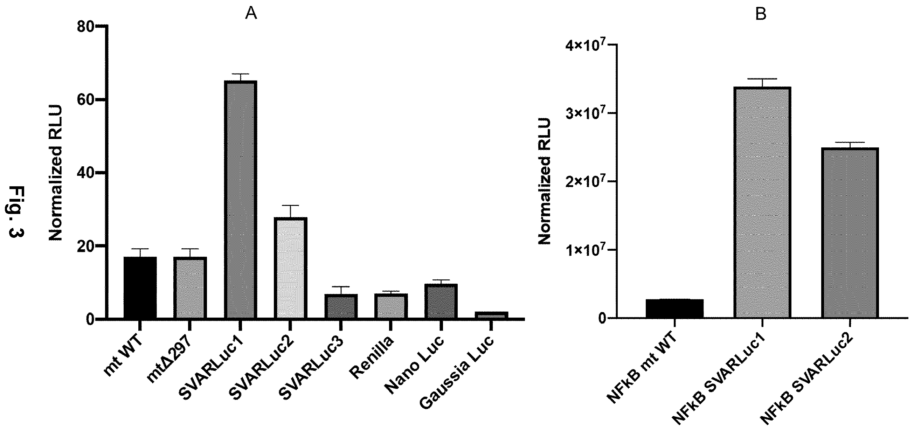

Fig. 3A illustrates the response of HEK293 cells following transient transfection of cells with the novel Svar vector encoding the luciferases genes indicated, each under the control of a constitutive CMV promoter, and co-transfection of cells with firefly luciferase under the control of a constitutive thymidine kinase (TK) promoter. Luciferase activity was quantified in the cell supernatant after 18 hours incubation of cells at 37°C using a commercially available coelenterazine based "glo" substrate (QuantiLuc Gold, Invivogen). The cells were then lysed and firefly luciferase activity was quantified with BrightGlo (Promega). Results are normalized relative to the expression of firefly luciferase that does not cross react with coelenterazine dependent luciferases.
Fig. 3B illustrates the response of HEK293 cells following transient transfection of cells with the novel Svar vector encoding either the wild type *Metridia longa* luciferase gene, or the Svar luciferase 1 or the Svar luciferase 2 genes each under the control of a chimeric promoter consisting of a SV40 minimal promoter and a 6-fold tandem repeat of the canonical NFkB recognition sequence and treated with 100 ng/ml of TNFα for 6 hours at 37°C prior to quantification of the luciferase signal using a commercially available coelenterazine based "glo" substrate (QuantiLuc Gold, Invivogen).

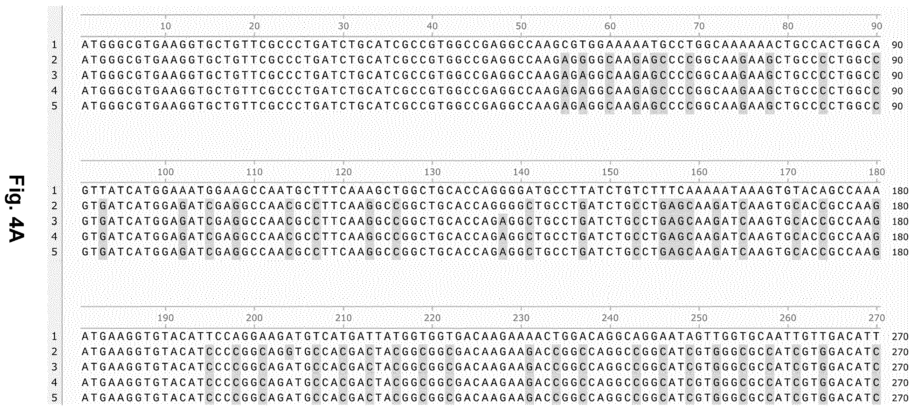

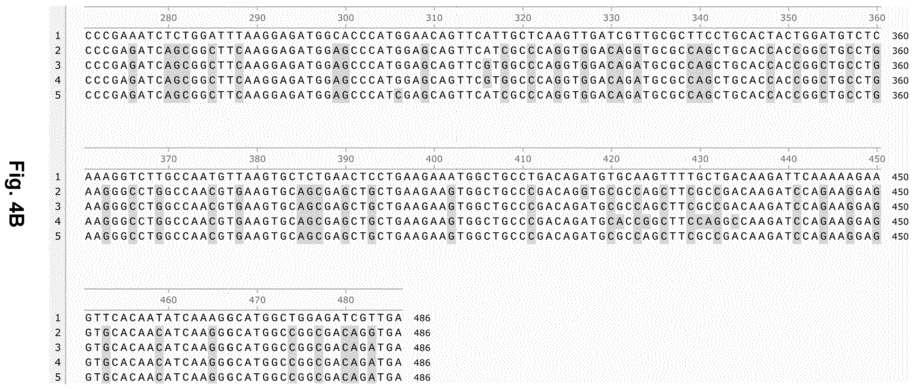

Fig. 4A & Fig. 4B illustrate a multiple sequence alignment of the coding sequence of the *Metridia longa* luciferase gene with a 177-nucleotide deletion in the 5' translated region of the gene and that of the four mutant luciferase genes. Nucleotides 1 to 51 in both the *Metridia longa* luciferase gene with a 177-nucleotide deletion in the 5' translated region and the four mutant luciferase genes represents the nucleotide sequence of the signal peptide of Gaussia luciferase.
   1. Nucleotide sequence of the coding region of the *Metridia longa* luciferase gene with a 177 nucleotide deletion in the 5' translated region
   2. Nucleotide sequence of the coding region of the SVAR Luc-1 luciferase gene.
   3. Nucleotide sequence of the coding region of the SVAR Luc-2 luciferase gene.
   4. Nucleotide sequence of the coding region of the SVAR Luc-3 luciferase gene.
   5. Nucleotide sequence of the coding region of the SVAR Luc-4 luciferase gene
Fig. 5 illustrate a multiple sequence alignment of the amino acid sequence of the *Metridia longa* luciferase gene with a 177-nucleotide deletion in the 5' translated region of the gene and that of the four mutant luciferase genes. Amino acids 1 to 17 in both the *Metridia longa* luciferase gene with a 177-nucleotide deletion in the 5' translated region and the four mutant luciferase proteins represents the amino acid sequence of the signal peptide of Gaussia luciferase.
   1. Amino acid sequence of the coding region of the *Metridia longa* luciferase gene with a 177 nucleotide deletion in the 5' translated region
   2. Amino acid sequence of the coding region of the SVAR Luc-1 luciferase gene.
   3. Amino acid sequence of the coding region of the SVAR Luc-2 luciferase gene
   4. Amino acid sequence of the coding region of the SVAR Luc-3 luciferase gene.
   5. Amino acid sequence of the coding region of the SVAR Luc-4 luciferase gene
Fig. 6A illustrates the rate of loss of luciferase activity in the supernatant of HEK293 cells following transient transfection of cells with the luciferases genes indicated, each under the control of a constitutive CMV promoter, and quantification of luciferase activity at increasing times at room temperature using a commercially available coelenterazine based "glo" substrate (QuantiLuc, Gold Invivogen).
Fig. 6B illustrates the relative luciferase activity at 15 minutes of the same samples shown in Fig. 6A.

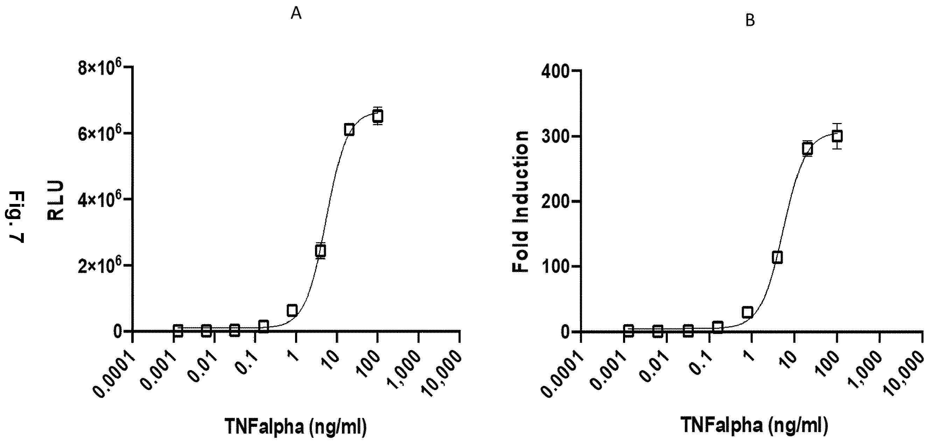

Fig. 7 illustrates the response of a clonal cell line of HEK293 cells stably transfected with a reporter gene comprising the sequence set forth in SEQ ID NO: 1 under the control of a chimeric promoter consisting of a SV40 minimal promoter and a 6-fold tandem repeat of the canonical NFkB recognition sequence treated with increasing concentrations of TNFα.
   Panel A. RLU values
   Panel B. Fold induction relative to the control not treated with TNFα
Fig. 8 illustrates a comparison of the response of K562 cells stably transfected with either the luciferase gene comprising the sequence set forth in SEQ ID NO: 1 under the control of a chimeric promoter consisting of a SV40 minimal promoter and a 6-fold tandem repeat of the canonical NFkB recognition sequence and K562 cells stably transfected with the firefly luciferase gene comprising the open reading frame encoding the firefly luciferase protein under the control of the same NFkB responsive promoter and treatment of cells with increasing concentrations of TNFα for 6 hours at 37°C.

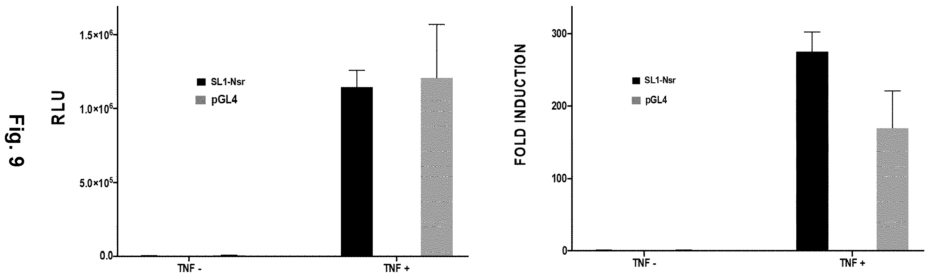

Fig. 9 illustrates the response of HEK293 cells following transient transfection of cells with either the plasmid expressing Svar luciferase 1 under the control of a chimeric promoter consisting of a SV40 minimal promoter and a 6-fold tandem repeat of the canonical NFkB recognition sequence or pGL4 (Promega) expressing firefly luciferase under the control of the same chimeric promoter and treated with 200 ng/ml of TNFα for 4 hours at 37°C prior to quantification of the Svar luciferase activity using a commercially available coelenterazine based "glo" substrate (QuantiLuc Plus, Invivogen) or quantification of firefly luciferase activity using Bright-Glo (Promega).

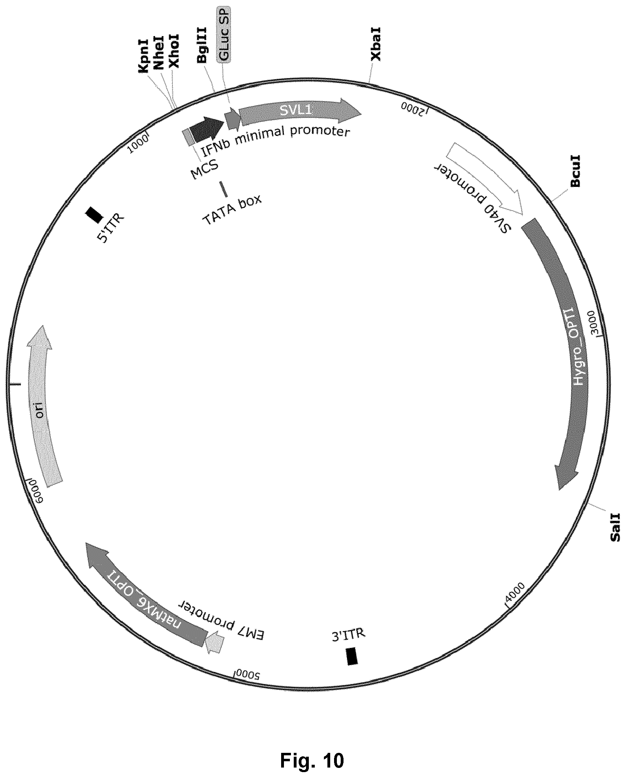

Fig. 10 shows the key features of the novel vector (6325 bp) comprising the codon optimized coding sequence of the Svar Luciferase-1 (SVL1) nt. 1330 to 1779, the Gaussia luciferase signal peptide nt. 1276 to 1329 (GLuc SP), the minimal promoter from the 5'-UTR of the human interferon beta gene nt.1141 to 1263, the transposon-specific 5' inverted terminal repeat nt. 652 to 682, the transposon-specific 3' inverted terminal repeat nt. 4567 to 4601, the codon optimized nourseothricine antibiotic resistance gene nt. 5138 to 5710 (natMx6_OPTI), under the control of a EM7 promoter nt. 5072-5137, a codon-optimized hygromycin resistance gene nt. 2532 to 3557 (Hygro OPTI), under the control of a SV40 minimal promoter nt. 2138 to 2495, and a multiple cloning site nt. 1110 to 1134 (MCS), and a bacterial origin of replication nt. 5954 to 217. Key restriction sites include; *Kpnl, Nhel, and Xho*l for the insertion of a consensus response sequence, *BcullSal*I for the substitution of a mammalian selection marker, *Bg*/II/Xbal for substitution of a reporter-gene, and XhoI/*Bgl*II for the substitution of a minimal promoter sequence.

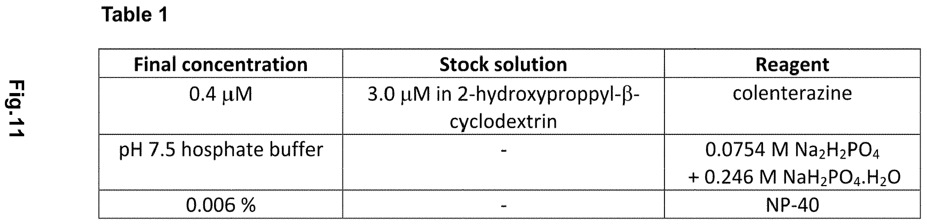

Fig. 11 illustrates Table1 showing the composition of a generic coelenterazine based luciferase substrate.

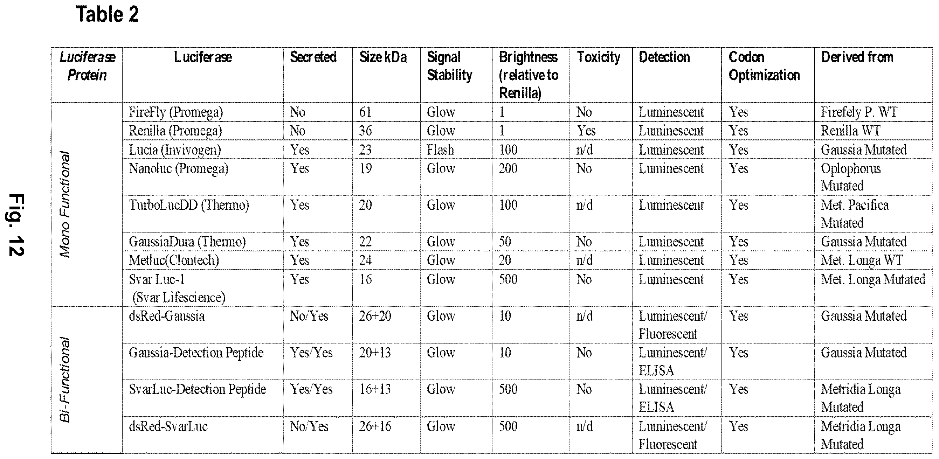

Fig. 12 illustrates Table 2 showing a comparison of the properties of the luciferase encoded by the sequence set forth in SEQ ID NO: 1 and SEQ ID NO:2 and comprising the amino acid sequence encoded by the sequence set forth in AA1 and AA2 relative to those of other native or engineered luciferases reported in the literature.

### Brief description of the nucleotide sequences

SEQ. ID NO:1. SVAR luciferase Luc-1, codon optimized
SEQ. ID NO:2. SVAR luciferase Luc-2, codon optimized
SEQ. ID NO:3. Gaussia signal peptide, codon optimized and encoding the Gaussia signal peptide.
SEQ. ID NO:4. Nourseothricine resistance gene, codon optimized
SEQ. ID NO:5. Whole nucleotide sequence of the SVAR plasmid
SEQ. ID NO:6. SVAR luciferase Luc-1, codon optimized, upstream of the nucleotide sequence of the F2A self-cleaving peptide and the nucleotide sequence of the firefly luciferase gene.

Nucleotides 1 to 54 Gaussia luciferase signal peptide
Nucleotides 55 to 501 Svar luciferase Luc-1, codon optimized
Nucleotides 517 to 582 encoding the F2A self-cleaving peptide
Nucleotides 582 to 2235 encoding the/a firefly luciferase, codon optimized

### Brief description of the amino acid sequences

SEQ. ID NO: AA1. SVAR luciferase Luc-1,encoded by SEQ ID NO: 1.
SEQ. ID NO: AA2. SVAR luciferase Luc-2, encoded by SEQ ID NO: 2.
SEQ. ID NO: AA3. Amino acid sequence of the Gaussia signal peptide
SEQ. ID NO: AA4. Amino acid sequence of the Gaussia signal peptide (amino acids 1 to 18) SVAR luciferase Luc-1, (amino-acids 19 to 167), F2A self-cleaving peptide (amino acids 168 to 194), firefly luciferase (amino-acids 195 to 745).

### Detailed description of the invention

In describing the embodiments of the invention specific terminology will be resorted to for the sake of clarity. However, the invention is not intended to be limited to the specific terms so selected, and it is understood that each specific term includes all technical equivalents which operate in a similar manner to accomplish a similar or equal purpose.

The present inventors provide i.a. for open reading frames, derived from the luciferase gene of *Metridia longa,* encoding luciferase proteins that have improved properties relative to the native protein and that have been codon-optimized for expression in mammalian cells and that exhibit optimal characteristics for use as a reporter gene for the quantification of the activity of pharmacology active molecules and methods of using the same for detecting/quantitating antibodies against pharmacology active molecules, and for high throughput screening in drug discovery.

In a particular embodiment, the invention relates to the open reading frame which may comprise or consist of a nucleotide sequence capable of encoding the amino acid sequence set forth in SEQ ID NO: AA1 or an amino acid sequence having a sequence identity of at least 75% to SEQ ID NO: AA1, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: AA1. In one aspect the nucleotide sequence is capable of encoding an amino acid sequence identical to the sequence set forth in SEQ ID NO: AA1.

In one aspect, SEQ ID NO: AA1 may be

MGVKVLFALICIAVAEAKRGKSPGKKLPLAVIMEIEANAFKAGCTRGCLICLS
KIKCTAKMKVYIPGRCHDYGGDKKTGQAGIVGAIVDIPEISGFKEMEPMEQF
IAQVDRCASCTTGCLKGLANVKCSELLKKWLPDRCASFADKIQKEVHNIKG
MAGDR.

In one aspect, the invention relates to the open reading frame which may comprise or consist of a nucleotide sequence set forth in SEQ ID NO: 1 encoding the amino acid sequence set forth in SEQ ID NO: AA1, or a nucleotide sequence having a sequence identity of at least 75% to SEQ ID NO:1, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: 1.

In a particular embodiment, the invention relates to the open reading frame comprises or consist of a nucleotide sequence capable of encoding the amino acid sequence set forth in SEQ ID NO: AA2 or an amino acid sequence having a sequence identity of at least 75% to SEQ ID NO: AA2, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: AA2. In one aspect the nucleotide sequence is capable of encoding an amino acid sequence identical to the sequence set forth in SEQ ID NO: AA2.

In one aspect, SEQ ID NO: AA2 may be

MGVKVLFALICIAVAEAKRGKSPGKKLPLAVIMEIEANAFKAGCTRGCLICLS
KIKCTAKMKVYIPGRCHDYGGDKKTGQAGIVGAIVDIPEISGFKEMEPMEQF
VAQVDRCASCTTGCLKGLANVKCSELLKKWLPDRCASFADKIQKEVHNIKG
MAGDR.

In one aspect, the invention relates to the nucleotide sequence set forth in SEQ ID NO: 2 encoding the amino acid sequence set forth in SEQ ID NO: AA2, or a nucleotide sequence having a sequence identity of at least 75% to SEQ ID NO:2, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: 2.

In another aspect, the invention relates to an open reading frame which may comprise or consist of a nucleotide sequence capable of encoding the amino acid sequence set forth in SEQ ID NO: AA1 and further comprise or consist of a nucleotide sequence capable of encoding the amino acid sequence set forth in SEQ ID NO: AA2.

In further aspects of the invention SEQ ID NO: AA3 may be MGVKVLFALICIAVAEAK, or a sequence having a sequence identity of at least 75% to SEQ ID NO:AA3, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: AA3.

In yet a further aspect of the invention SEQ ID NO: AA4 may be 
MEAEAERGKSPGKKLPLAVIMEIEANAFKAGCTRGCLICLSKIKCTAKMKVYI
PGRCHDYGGDKKTGQAGIVGAIVDIPEISGFKEMEPMEQFIAQVDRCASCT
TGCLKGLANVKCSELLKKWLPDRCASFADKIQKEVHNIKGMAGDRKIVAPV
KQTLNFDLLKLAGDVESNPGPMEDAKNIKKGPAPFYPLEDGTAGEQLHKA
MKRYALVPGTIAFTDAHIEVDITYAEYFEMSVRLAEAMKRYGLNTNHRIVVC
SENSLQFFMPVLGALFIGVAVAPANDIYNERELLNSMGISQPTVVFVSKKGL
QKILNVQKKLPIIQKIIIMDSKTDYQGFQSMYTFVTSHLPPGFNEYDFVPESF
DRDKTIALIMNSSGSTGLPKGVALPHRTACVRFSHARDPIFGNQIIPDTAILS
VVPFHHGFGMFTTLGYLICGFRVVLMYRFEEELFLRSLQDYKIQSALLVPTL
FSFFAKSTLIDKYDLSNLHEIASGGAPLSKEVGEAVAKRFHLPGIRQGYGLT
ETTSAILITPEGDDKPGAVGKVVPFFEAKVVDLDTGKTLGVNQRGELCVRG
PMIMSGYVNNPEATNALIDKDGWLHSGDIAYWDEDEHFFIVDRLKSLIKYKG
YQVAPAELESILLQHPNIFDAGVAGLPDDDAGELPAAVVVLEHGKTMTEKEI
VDYVASQVTTAKKLRGGVVFVDEVPKGLTGKLDARKIREILIKAKKGGKIAV*,
 or a sequence having a sequence identity of at least 75% to SEQ ID NO:AA4, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: AA4.

In another aspect, present invention relates to a cell or cell line. The cell or dell line according to the invention, wherein said cell may comprises a polynucleotide comprising promoter operably linked to a down-stream open reading frame encoding at least one amino acid according to the invention.

The cell or cell line may comprise an open reading frame which may comprise or consist of a nucleotide sequence capable of encoding the amino acid sequence set forth in SEQ ID NO: AA1 or an amino acid sequence having a sequence identity of at least 75% to SEQ ID NO: AA1, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: AA1. In one aspect the nucleotide sequence is capable of encoding an amino acid sequence identical to the sequence set forth in SEQ ID NO: AA1.

In another aspect, the cell or cell line according to the invention may comprise an open reading frame which comprises or consist of a nucleotide sequence capable of encoding the amino acid sequence set forth in SEQ ID NO: AA2 or an amino acid sequence having a sequence identity of at least 75% to SEQ ID NO: AA2, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: AA2. In one aspect the nucleotide sequence is capable of encoding an amino acid sequence identical to the sequence set forth in SEQ ID NO: AA2.

In another aspect, the cell or cell line may comprise an open reading frame which may comprise or consist of a nucleotide sequence capable of encoding the amino acid sequence set forth in SEQ ID NO: AA1 or an amino acid sequence having a sequence identity of at least 75% to SEQ ID NO: AA1, and an open reading frame which comprises or consist of a nucleotide sequence capable of encoding the amino acid sequence set forth in SEQ ID NO: AA2 or an amino acid sequence having a sequence identity of at least 75% to SEQ ID NO: AA2. In one non-limiting example, the cell or cell line may comprise SEQ ID NO:1 encoding SEQ ID NO: AA1, and SEQ ID NO:2 encoding SEQ ID NO: AA2.

Present invention also relates to one or more of SEQ ID NO: 3-6, or any such sequence having a sequence identity of at least 75% to SEQ ID NO: 3-6, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: 3-6.

In another aspect, present invention relates to a cell or cell line which may further comprise or consist of an open reading frame which may comprise or consist of a nucleotide sequence as set forth in SEQ ID NO:6, or any such sequence having a sequence identity of at least 75% to SEQ ID NO: 6, such as e.g. at least 80% sequence identity, such as e.g. at least 85% sequence identity, such as e.g. at least 90% sequence identity, such as e.g. at least 95% sequence identity, such as e.g. at least 96% sequence identity, such as e.g. at least 97% sequence identity, such as e.g. at least 98% sequence identity, such as e.g. at least 99% sequence identity, or such as e.g. 100% sequence identity to the sequence as set forth in SEQ ID NO: 6. Thus a cell or cell line according to the invention may comprise one or SEQ ID NO:1 or 2 and additionally comprise SEQ ID NO: 6.

In a further aspect, present invention relates to a cell or cell line which may comprise or consist of an open reading frame which may comprise or consist of at least one or more of nucleotide sequence as set forth in SEQ ID NO:1-4.

In one aspect, the invention relates to a cell or cell line which may comprise or consist of an open reading frame which may comprise or consist of a nucleotide sequence as set forth in SEQ ID NO:1 and may further comprise one or more of SEQ ID: 3-4, wherein the entire collected sequence may be denoted SEQ ID NO.: 5.

In another aspect, the invention relates to a cell or cell line which may comprise or consist of an open reading frame which may comprise or consist of a nucleotide sequence as set forth in SEQ ID NO:2 and may further comprise one or more of SEQ ID: 3-4.

In one aspect, present invention relates to use of an amino acid sequence, or a polynucleotide capable of encoding any one of the amino acids sequences of present invention, or a cell or cell line in any diagnostic method.

In one aspect, the invention relates to use of an amino acid sequence, or a polynucleotide capable of encoding any one of the amino acids sequences of present invention, or a cell or cell line for determining the presence of neutralizing antibodies in a biological sample.

In another aspect, present invention relates to use an amino acid sequence, or a polynucleotide capable of encoding any one of the amino acids sequences of present invention, or a cell or cell line in drug discovery, such as e.g. use in screening of drugs or drug candidates. In a particular aspect, the screening may be relates to high through-put screening.

Consequently, present invention also relates to a method for detecting and optionally quantitating the activity of a pharmacology active molecule in a test sample. In one aspect, said method comprising may comprise the steps of;
(i) providing a test sample,
(ii) contacting said test sample with the cell line according to the present invention, said cell line containing a first heterologous polynucleotide comprising a heterologous cis-acting regulatory sequence, that responds to treatment of the cell line with the pharmacology active molecule(s) present in the test sample, operably linked to a downstream promoter sequence, wherein said promoter is operably linked to an open reading frame comprising one of the sequences set forth in SEQ ID NO: 1 and SEQ ID NO: 2 encoding a first reporter protein,
(iii) Optionally, providing for a normalization of the assay, employing the cell lines according to the present invention further having a construct for the constitutive production of a luciferase that is different from that used in the reporter gene construct that responds to a pharmacology active molecule in (ii). In one apsect, the constitutive production may be of a second luciferase which is different frpom the reporter protein of (ii), may in principle be any suitable reporter protein and may be e.g., Renilla luciferase or firefly luciferase.
(iv) determining the activity of the first reporter protein (in (ii)) in said cell line using a colenterazine based substrate such as that the composition of which is shown in Table 1 or a commercially available substrate such as Quanti-Luc Gold (InvivoGen), or another commercially available coelenterazine based substrate.
(v) determining the activity of the second reporter protein (in (iii)) in said cell line after the reporter gene luciferase is measured in the same sample using the Stop & Glo reagent from the Dual-Glo system (Promega), or a detection system comprising a second component of the dual luciferase buffer that quenches firefly luciferase activity and contains a coelenterazine based substrate allowing subsequent quantification of Renilla luciferase activity, and that efficiently inhibits the activity of the first reporter protein encoded by one the sequences set forth in SEQ ID NO: 1 and SEQ ID NO: 2 in the absence of colenterazine.
(vi) The activity of the first luciferase normalized relative to the activity of the second luciferase is described in US 2011/0189658 and incorporated herein by reference in its entirety.

In a further preferred embodiment, to provide for a dual-luciferase assay using different substrates, the cells according to the present invention may further have a dual or poly-cistronic construct operably linked to an open reading frame encoding a luciferase reporter protein, wherein said open reading frame comprises or consist of the nucleotide sequence set forth in SEQ ID NO: 1 or SEQ ID NO: 2 and a second open reading frame encoding firefly luciferase or any other luciferase that uses luciferin as a substrate separated by the coding sequence of a self-cleaving 2A peptide F2A set forth in SEQ ID NO: 6.

The method according to the invention may also alternatively comprise the steps :
(vii) determining the activity of the first reporter protein in said cell line using a coelenterazine based substrate such as that the composition of which is shown in Table 1 or a commercially available substrate such as Quanti-Luc Gold (InvivoGen), or another commercially available coelenterazine based substrate.
(viii) determining the activity of the second reporter protein in said cell line after the reporter gene luciferase is measured in the same sample using the BrightGlo reagent (Promega), or a commercially available luciferin based substrate.

In a further preferred embodiment, to provide for a tri-luciferase assay using different substrates, the cells according to the present invention further have 3 constructs each operably linked to different ligand-responsive promoter operationally linked an open reading frame encoding a luciferase reporter protein, wherein said open reading frame comprises or consist of the nucleotide sequence set forth in SEQ ID NO: 1 or SEQ ID NO: 2 and a second open reading frame encoding firefly luciferase or any other luciferase that uses luciferin as a substrate together with third open reading frame encoding Renilla luciferase or any other luciferase that uses coelenterazine as a substrate. Alternatively, Renilla luciferase may be operationally linked to a constitutive promoter to allow the activity of the two ligand responsive luciferase to be normalized relative to the activity of a luciferase under the control of a constitutive promoter described in US 2011/0189658 incorporated herein by reference in its entirety.

The method according to the invention may alternatively comprise the steps of;
(ix) determining the activity of the first reporter protein wherein said open reading frame comprises or consist of the nucleotide sequence set forth in SEQ ID NO: 1 or SEQ ID NO: 2 under the control of a signal peptide that ensures efficient secretion of the first reporter protein into the culture medium or supernatant of said cell line using a coelenterazine based substrate such as that the composition of which is shown in Table 1 or a commercially available substrate such as Quanti-Luc Gold (InvivoGen), or another commercially available coelenterazine based substrate.
(x) determining the activity of the second reporter protein requiring a luciferin based substrate such as firefly luciferase in said cell line following cell lysis using the DualGlo reagent (Promega), using a commercially available two component substrate the first component of which is a luciferin-based substrate that allows the substrate that allows the quantification of firefly luciferase activity and a second component that quenches firefly luciferase activity and contains a coelenterazine based substrate allowing quantification of Renilla luciferase activity after which the activity of the third reporter gene such as Renilla luciferase that requires a coeleranterize based substrate is measured in the same sample using the DualGlo reagent (Promega), or another commercially available equivalent.

As mentioned above, the present invention also concerns a method for high throughput screening in drug discovery said method comprising the steps of
(i) providing a test sample(s), consisting of a library of the compounds to be screened
(ii) contacting said test sample(s) with the cell line according to the present invention, said cell line containing a first heterologous polynucleotide comprising a heterologous cis-acting regulatory sequence, that responds to treatment of the cell line with the pharmacology active molecule(s) present in the test sample(s), operably linked to a downstream promoter sequence, wherein said promoter is operably linked to an open reading frame encoding a first reporter protein comprising one the sequences set forth in SEQ ID NO: 1 and SEQ ID NO: 2. In one aspect, the cell line according to the present invention, may be seeded in 96, 384 or 1536 well plates assay plates.
(iii) Treatment of the cell line according to the present invention with the agent(s) or drug candidates used to screen the library.
(iv) Optionally, to provide for a normalization of the assay, the cell lines according to the present invention further have a construct for the constitutive production of a luciferase that is different from that used in the reporter gene construct and thus different from the reporter proteins encoded by SEQ ID NO:1 or SEQ ID NO:2. For example, the constitutive production may be of a second luciferase, e.g., Renilla luciferase or firefly luciferase.
(v) determining the activity of the first reporter protein in said cell line using a coelenterazine based substrate such as that the composition of which is shown in Table 1 or a commercially available substrate such as Quanti-Luc Gold (InvivoGen), or another commercially available coelenterazine based substrate.
(vi) determining the activity of the second reporter protein in said cell line after the reporter gene luciferase is measured in the same sample using Bright Glo (Promega), or another commercially available luciferin based substrate that efficiently inhibits the activity of the first reporter protein comprising one the sequences set forth in SEQ ID NO: 1 and SEQ ID NO: 2 in the absence of coelenterazine.
(vii) Determining the activity of the first luciferase normalized relative to the activity of the second luciferase is described in US 2011/0189658 incorporated herein by reference in its entirety.

A further aspect of the present invention concerns a method detecting and optionally quantitating neutralizing antibodies against a pharmacology active molecule(s) present in the test sample.

The method according to the invention may comprising the steps of;
(i) providing a test sample comprising a pharmacology active molecule(s),
(ii) providing a first and a second cell sample, wherein said cells samples comprises the cell line according to the present invention
(iii) contacting the first cell sample in (ii) with the test sample (in (i)) and subsequently with the pharmacology active molecule,
(iv) contacting the second cell sample with the pharmacology active molecule,
(iv) determining the activity of the first reporter protein in the cells of the first cell sample and determining the activity of the first reporter protein in the cells of the second cell sample,
(v) provide the ratio between the reporter activity in first and a second cell sample, where a ratio (first/second) lower than one is indicative for the presence of antibodies against the pharmacology active molecule in said sample.

### Definitions

### Vector

The term "vector" or vector construct" refers to a DNA molecule used as a vehicle to transfer recombinant genetic material into a host cell. The four major types of vectors are plasmids, bacteriophages and other viruses, cosmids, and artificial chromosomes. The vector itself is generally a DNA sequence that consists of an insert (a heterologous nucleic acid sequence, transgene) and a larger sequence that serves as the "backbone" of the vector. The purpose of a vector which transfers genetic information to the host is typically to isolate, multiply, or express the insert in the target cell. Vectors called expression vectors (expression constructs) are specifically adapted for the expression of the heterologous sequences in the target cell, and generally have a promoter sequence that drives expression of the heterologous sequences. The choice of vector employed in embodiments of the present invention depends on the specific application of the vector encoding the polypeptides or polynucleotide.

### Operatively linked

The term "operatively linked" refers to the connection of elements being a part of a functional unit such as a gene or an open reading frame. Accordingly, by operatively linking a promoter to a nucleic acid sequence encoding a polypeptide (an open reading frame, ORF) the two elements become part of the functional unit - a gene. The linking of the expression control sequence (promoter) to the nucleic acid sequence enables the transcription of the nucleic acid sequence directed by the promoter. By operatively linking two heterologous nucleic acid sequences encoding a polypeptide the sequences become part of the functional unit - an open reading frame encoding a fusion protein comprising the amino acid sequences encoding by the heterologous nucleic acid sequences. By operatively linking two amino acids sequences, the sequences become part of the same functional unit - a polypeptide. Operatively linking two heterologous amino acid sequences generates a hybrid (fusion) polypeptide.

### Minimal constitutive promoter

The promoter directing the expression of a reporter gene is typically minimally constitutively active in the mammalian host cell used to establish the reporter cell line of the present invention and alone does not respond to treatment of cells with a pharmacologically active molecule. Useful constitutively active promoters include but at not limited to cytomegalovirus (CMV) early enhancer/promoter, SV40 promoter, UBC promoter, PGK promoter, human β-actin (hACTB), human elongation factor-1α (hEF-1α), Thymidine Kinase (TK) promoter and cytomegalovirus early enhancer/chicken β-actin (CAG) promoters.

When describing the embodiments of the present invention, the combinations and permutations of all possible embodiments have not been explicitly described. Nevertheless, the mere fact that certain measures are recited in mutually different dependent claims or described in different embodiments does not indicate that a combination of these measures cannot be used to advantage. The present invention envisages all possible combinations and permutations of the described embodiments.

The terms "comprising", "comprise" and "comprises" herein are intended to be optionally substitutable with the terms "consisting of", "consist of" and "consist of", respectively, in every instance. The invention will be more fully understood by reference to the detailed description of the invention. This should not, however, be construed as limiting the scope of the invention. All literature citations are incorporated herein by reference in their entirety.

### Examples

In the following, the invention is further illustrated by non-limiting examples.

To develop a luciferase protein that exhibits optimal characteristics for use as a reporter gene for the quantification of the activity of pharmacology active molecules, and for high throughput screening in drug discovery it is desirable that the gene is as small as possible to facilitate expression in mammalian cells particularly when the luciferase is expressed as a fusion protein with other proteins or other molecules. A series of deletion mutants of the 5' coding region of the native *Metridia longa* luciferase gene were designed *in silico,* synthetized *in vitro* and tested in transient transfection experiments. The nucleotide sequence of the native *Metridia longa* gene luciferase gene is illustrated in Fig. 1 and the amino acid sequence in Fig. 2. The deletion mutant that comprises amino acid 1 from the initiation codon (AUG) to amino acid 76 in the 5' translated region of the native *Metridia longa* luciferase gene (Fig. 1) was found to exhibit an activity comparable to that of the native protein (Fig. 3) while reducing the size of the luciferase protein by 76 amino acids equivalent to a reduction in molecular weight of 6.5 kDa given that the molecular weight of the SVAR Luc-1 protein is 17.3 kDa compared with a molecular weight of 23.8 kDa for the native Metridia longa protein. To increase the efficiency of secretion, and hence reduce background levels of bioluminescence and to allow serial sampling, the signal peptide of the luciferase gene from the calanoid copepod Gaussia that has been shown to be the most efficient signal peptide described to date (Stern, B., et al, 2007) was substituted for the native signal peptide (nt. 1 to nt. 51) of the *Metridia longa* luciferase gene (Fig. 1) encoding the 17 amino acids of the signal peptide (Fig. 2). To enhance the efficiency of secretion and reduce background levels in cells transfected with a reporter gene construct, that responds to treatment of cells with a pharmacology active molecule, hence increasing the signal relative to control cells not treated with a pharmacology active molecule the native signal peptide of the *Metridia longa* luciferase gene was deleted and replaced with the codon optimized signal peptide from the gene encoding Gaussia luciferase.

A series of random mutation were introduced into the coding sequence of the native *Metridia longa* luciferase gene using oligonucleotides carrying a single or multiple mutations (Fig. 4). The oligonucleotides were synthetized *in vitro* and tested in transient transfection experiments in human the HEK293 cell line originally derived from human embryonic kidney cells grown in tissue culture (ATCC Catalogue CRL-1573). Specific mutations introduced into the coding sequence of the native *Metridia longa* luciferase gene were found both individually and together to encode a luciferase protein that is superior to that encoded by the native gene in at least one of the following characteristics; enhanced luminescence including increased light output (brightness), enhanced signal stability and/or signal duration as reflected by high relative light units (RLU), and increased dynamic range relative to control samples in the absence of the pharmacology active substance. Mutation of the nucleotides encoding the methionine's at positions 66 and 105 into serine (S) and isoleucine (I) respectively, in the first putative catalytic domain (Markova, SV., et al., 2004) as shown in Fig. 5, was found to stabilize light emission allowing the use of a "glo" substrate and obviating the need for a luminometer equipped with injectors.

Mutation of the alanine (A) at position 100 in the putative second catalytic domain (Markova, SV., et al., 2004) of the amino acid sequence encoded by the native *Metridia longa* luciferase gene into glutamic acid (E) was found to encode luciferase proteins (SVAR Luc-1 and SVAR Luc-2) that exhibited an increased luminescence including increased light output (brightness), increased signal stability and/or signal duration as reflected by higher relative light units (RLU) than that exhibited by the native gene or the gene encoding the mutations at positions 22 and 35 only in the amino acid sequence (Fig. 5), and a higher dynamic range relative to control samples in the absence of the pharmacology active substance than that exhibited by the native *Metridia longa* luciferase gene (Fig. 3B).

Mutation of the isoleucine (I) at position 106 in the amino acid sequence into a valine (V) in the first putative catalytic domain was found to result in a luciferase protein SVAR Luc-2 (nucleotide SEQ ID NO: 2 and amino sequence SEQID NO: AA2) that exhibited an improved activity relative to the native *Metridia longa* luciferase or the luciferase encoded by the *Metridia longa* luciferase gene with a 177 nucleotide deletion in the 5' translated region and an equivalent or slightly reduced activity relative to SVAR Luc-1 luciferase (nucleotide SEQ ID NO: 1 and amino sequence SEQ ID NO: AA1).

Mutation of the sequence nt 423 to nt 435 in the coding sequence of the native *Metridia longa* luciferase gene encoding the amino acids alanine (A) and serine (S) at positions 141 and 142 to amino acids tyrosine (T) and glycine (G) respectively was found to encode a luciferase protein (SVAR Luc-3) that exhibited decreased luminescence including decreased light output (brightness), decreased signal stability and/or signal duration as reflected by lower relative light units (RLU) than that exhibited by the native gene, and a lower dynamic range relative to control samples in the absence of the pharmacology active substance than that exhibited by the native *Metridia longa* luciferase gene, Svar luciferase 1 or Svar luciferase 2 (Fig. 3). Similarly, mutation of nt 432 and nt 433 encoding the alanine (A) at position 144 and the methionine at position 145 respectively in the amino acid sequence encoded by the native *Metridia longa* luciferase gene was found to encode a luciferase protein (SVAR Luc-4) that exhibited decreased luminescence including decreased light output (brightness), decreased signal stability and/or signal duration as reflected by lower relative light units (RLU) than that exhibited by the native *Metridia longa* luciferase gene, Svar luciferase Luc-1 or Svar luciferase Luc-2 (Fig. 3B). Both Svar luciferases 1 and Svar luciferases 2 under the control of a strong constitutive CMV promoter tested in transient transfection experiments in human HEK293 cells for 18 hours at 37°C prior to the quantification of luciferase activity using a commercially available coelenterazine based "glo" substrate QuantiLuc Gold (Invivogen) exhibited increased signal stability and/or signal duration than that exhibited by the wild type *Metridia longa* luciferase (Fig. 6). Both Svar luciferases 1 and Svar luciferases 2 also exhibited increased light output (brightness), as reflected by higher relative light units (RLU) than that exhibited by the native *Metridia longa* gene, or the *Metridia longa* gene with the deletion of amino acids 1 to 76 in the 5' translated region of the gene, or SVAR luciferase 3, or Renilla luciferase, Nano luciferase, or Gaussia luciferase (Fig. 3A). The results of transient transfection experiments in HEK293 cells transfected with the wild type *Metridia longa* luciferase gene, the Svar luciferase 1 or the Svar luciferase 2 each under the control of a chimeric promoter consisting a SV40 minimal promoter and a 6-fold tandem repeat of the canonical NFkB recognition sequence and treated or not with 100 ng/ml of TNFα for 6 hours at 37°C showed that both SVAR luciferase genes exhibited enhanced luciferase activity relative to the wild type gene as reflected by a higher relative light units (RLU) using a commercially available coelenterazine based "glo" substrate QuantiLuc Plus (Invivogen) (Fig. 3B).

HEK293 cells were transfected with the Svar luciferase Luc-1 gene under the control of a chimeric promoter consisting a SV40 minimal promoter and a 6-fold tandem repeat of the canonical NFkB recognition sequence and stable clones were isolated and characterized. Treatment of one such clonal cell line or not with increasing of TNFα for 6 hours at 37°C resulted in high levels of both relative light units (RLU) and fold induction relative to the control sample not treated with TNFα (Fig. 7) using a commercially available coelenterazine based "glo" substrate QuantiLuc Gold (Invivogen).

K562 cells originally derived from a chronic myelogenous leukemia (ATCC, catalogue number CCL-243) were transfected with either the Svar luciferase Luc-1 gene or firefly luciferase gene each under the control of a chimeric promoter consisting a SV40 minimal promoter and a 6-fold tandem repeat of the canonical NFkB recognition sequence. Stable clones were isolated and characterized. The clonal cell lines were treated with increasing concentrations of TNFα for 6 hours at 37°C (Fig. 8). Cells stably transfected with the SVAR Luc-1 gene exhibited markedly higher relative light units (RLU) values than cells stably transfected with the firefly luciferase gene under the control of the same NFkB responsive promoter following quantification of luciferase activity using a commercially available coelenterazine based "glo" substrate QuantiLuc Gold (Invivogen) (Fig. 8).

The SVAR Luc-1 luciferase presents several improved properties relative to other native or engineered luciferases reported in the literature including small size, brightness, and absence of toxicity for transfected mammalian cells (Table 2).

To develop a plasmid that exhibits optimal characteristics for use as a vector for the expression of the Svar Luc-1 and Svar Luc-2 luciferases as a reporter gene, including but not limited to, the nucleotide sequences set forth in SEQ ID NO: 1 and SEQ ID NO: 2, for the quantification of the activity of pharmacology active molecules, and for high throughput screening in drug discovery it is desirable that the plasmid can be replicated readily extra chromosomally in laboratory strains of *E*. *coli,* and contains the necessary DNA sequences required for this purpose, and is relatively small in size, of the order of 3,000 to 6,000 base-pairs such that there is a lower probability of interference with host factors resulting in an increased yield in bacteria. Thus, a plasmid (pSVAR001) was designed *in silico* and synthetized *in vitro* containing: a bacterial origin of replication (nt. 5954 to 217), a minimal promoter from the 5' UTR of the human interferon beta gene (nt. 1141-1263), the signal peptide from the Gaussia luciferase gene (nt. 1276-1329) that has been codon optimized (SEQ. ID NO 3), the coding sequence of a reporter-gene (nt. 1330-1779), including but not limited to, the nucleotide sequences set forth in SEQ ID NO: 1 and SEQ ID NO: 2, and the transposon-specific 5' (nt. 652-682) and 3' (nt. 4567-4601) inverted terminal repeats, the recognition sequences for a transposase integration of a transgene, the nourseothricine antibiotic resistance gene (nt. 5138-5710) under the control of a EM7 promoter (nt. 5072-5137), and a codon-optimized hygromycin resistance gene (nt. 2532-3557), under the control of a SV40 minimal promoter (nt. 2138-2495), that comprises or consist of the sequence set forth in SEQ ID NO: 4. The plasmid further contains a number of key restriction sites: Kpnl, Nhel, and Xhol for the insertion of a consensus response sequence, Bcul-Sall for the substitution of a mammalian selection marker, Bgll-Xbal for the substitution of a reporter-gene including but not limited to the sequences set forth in SEQ ID NO: 1 and SEQ ID NO: 2, and XhoI-BgllI for the substitution of a minimal promoter sequence. To test the performance of the pSVAR001 plasmid a 6-fold tandem repeat of the canonical NFkB recognition sequence was inserted into the Kpn-Xhol restriction site and the resulting plasmid was tested in transient transfection experiments in human HEK293 cells treated or not with 200 ng/ml of TNFα for 4 hours at 37°C prior to the quantification of SVAR1 luciferase activity using a commercially available coelenterazine based substrate (QuantiLuc Gold, Invivogen). A comparison of the performance of the pSVAR001 plasmid containing a 6-fold tandem repeat of the canonical NFkB recognition sequence with that of the pGL4 plasmid (Promega) containing the SVAR Luc-1 luciferase gene inserted into the BglII-XbaI restriction site under the control of the 6-fold tandem repeat of the canonical NFkB recognition sequence inserted into a Kpn-Xhol restriction site showed that maximum RLU levels obtained with the pSVAR001 plasmid were comparable to those obtained with the pGL4 plasmid containing the SVAR Luc-1 luciferase gene under the control of the 6-fold tandem repeat of the canonical NFkB recognition sequence while fold induction was significantly greater using the pSVAR001 plasmid (Fig. 9).

### References

Inglese, J., et al., Nat. Chem. Biol. 3:466-479, 2007
Thorne, N., et al., Chem. Biol. 17:646-657, 2010
Markova, SV., et al., J. Biol. Chem. 279:3212-3217, 2004
Stern, B., et al. Trends Cell Mol. Biol. 2:1-17, 2007
Corish, P., and Tyler-Smith, C., Protein Eng., 12:1035-1040, 1999
Lallemand et al. J. Interferon & Cytokine Res. 28:393-404, 2008.
Lallemand et al. J.Immunol. Methods. 356: 18-28, 2010.
Lallemand et al. J.Immunol. Methods. 373: 229-239, 2011
Lallemand et al. J.Immunol. Res. 390: 1-19, 2017

In specific embodiments, present invention also relates to the following items:
1. A metazoan cell comprising
   (i) a first heterologous polynucleotide comprising a heterologous cis-acting regulatory sequence operably linked to a downstream promoter sequence, wherein said promoter is operably linked to an open reading frame encoding a luciferase reporter protein, wherein said open reading frame comprises or consist of the nucleotide sequence set forth in SEQ ID NO: 1 or SEQ ID NO: 2.
   (ii) In a preferred embodiment, to provide for a normalization of the assay, the cells according to the present invention further have a construct for the constitutive expression of a luciferase that is different from that used in the reporter gene construct that responds to a pharmacology active molecule. For example, the constitutive production may be of a second luciferase, e.g., Renilla luciferase or firefly luciferase under the control of a constitutive promoter.
   (xi) In a further preferred embodiment, to provide for a tri-luciferase assay using different substrates, the cells according to the present invention further have dual constructs each operably linked to a different ligand-responsive promoter and operably linked an open reading frame encoding a luciferase reporter protein, wherein said open reading frame comprises or consist of the nucleotide sequence set forth in SEQ ID NO: 1 or SEQ ID NO: 2 and a second open reading frame encoding firefly luciferase or any other luciferase that uses luciferin as a substrate together with third open reading frame encoding Renilla luciferase or any other luciferase that uses coelenterazine as a substrate under the control of either a third ligand-responsive promoter or under the control of a constitutive promoter to allow the activity of the two ligand-responsive luciferase to be normalized relative to the activity of a luciferase under the control of a constitutive promoter described in US 2011/0189658.
   (iii) determining the activity of the first reporter protein wherein said open reading frame comprises or consist of the nucleotide sequence set forth in SEQ ID NO: 1 or SEQ ID NO: 2 under the control of a signal peptide that ensures efficient secretion of the first reporter protein in the culture medium or supernatant of said cell line using a coelenterazine based substrate such as that the composition of which is shown in Table 1 or a commercially available substrate such as Quanti-Luc Gold (InvivoGen).
   (iv) determining the activity of the second reporter protein requiring a luciferin based substrate such as firefly luciferase in said cell line after lysis of the cells using the Dual-Glo substrate (Promega) which the activity of the third reporter gene such as Renilla luciferase that requires a coeleranterize based substrate is measured in the same sample following cell lysis using the DualGlo reagent (Promega) that efficiently inhibits the activity of the second reporter protein prior to quantification of the activity of the third reporter-gene.
2. The mammalian cell according to the preceding item, wherein said open reading frame comprises or consist of the sequence set forth in SEQ ID NO: 1 or SEQ ID NO: 2 or a sequence having at least 75% sequence identity to SEQ ID NO: 1, or SEQ ID NO: 2 such as 79% sequence identity to SEQ ID NO: 1 or SEQ ID NO: 2, for example 85% sequence identity to SEQ ID NO: 1 or SEQ ID NO: 2, such as 90% sequence identity to SEQ ID NO: 1 or SEQ ID NO: 2, such as 95% sequence identity to SEQ ID NO: 1 or SEQ ID NO: 2, for example 97% sequence identity to SEQ ID NO: 1 or SEQ ID NO: 2, such as 98% sequence identity to SEQ ID NO: 1 or SEQ ID NO: 2, for example 99% sequence identity to SEQ ID NO: 1 or SEQ ID NO: 2.10. The mammalian cell according to any of the preceding claims, wherein said open reading frame comprises or consist of the sequence set forth in SEQ ID NO: 1.
3. The cell according to any of the preceding items, wherein said cell is selected from the group consisting of but not limited to HEK293, HEK293T, K562, U937, Jurkat, Molt-4, HeLa cells, HT1080 cells, ARPE-19, ARPE-19/HPV-16, insect cell such as Sf9 cells, avian cells such as DT-40, MSB1, or LMH, mouse cells such as L929 cells or the LS variant, Chinese hamster ovary (CHO) cells such as CHO-K1, CHO-DXB11, CHO-DG44, CHOK1SV cells including all variants.
4. The cell line according to any of the preceding items, wherein said cell line is HEK293.
5. A method for detecting and optionally quantitating the activity of a pharmacologically active molecule in a test sample, said method comprising the steps of
   (i) providing a test sample,
   (ii) contacting said test sample with the cell line according to any one of the preceding items,
   (iii) determining the activity of the first reporter protein in said cell line.
6. The method according to any of items, wherein said cell line expresses a second reporter protein, said method further comprising (iv) determining the activity of the first reporter protein in said cell line, (v) providing the ratio between the activity of the first reporter protein and the second reporter protein.
7. A method for high throughput screening in drug discovery said method comprising the steps of:
   (i) providing a test sample(s), consisting of a library of the compounds to be screened
   (ii) contacting said test sample(s) with the cell line according to the present invention, said cell line containing a first heterologous polynucleotide comprising a heterologous cis-acting regulatory sequence, that responds to treatment of the cell line with the pharmacology active molecule(s) present in the test sample(s), operably linked to a downstream promoter sequence, wherein said promoter is operably linked to an open reading frame encoding a first reporter protein comprising one the sequences set forth in SEQ ID NO: 1 and SEQ ID NO: 2. In a preferred embodiment the cell line according to the present invention, is seeded in 384 or 1536 well plates assay plates.
8. A plasmid that exhibits optimal characteristics for use as a vector for the expression of a reporter gene for the quantification of the activity of pharmacology active molecules.
9. A plasmid that exhibits optimal characteristics for use as a vector for the expression of a reporter gene wherein said reporter gene comprises or consist of the nucleotide sequence set forth in SEQ ID NO: 1 or SEQ ID NO: 2.
10. A plasmid that exhibits optimal characteristics for use as a vector for the expression of a reporter gene in mammalian cells containing a codon optimized hygromycin resistance gene that comprises or consist of the nucleotide sequence set forth in SEQ ID NO: 4
11.A plasmid that exhibits optimal characteristics for use as a vector for the expression of a reporter gene in mammalian cells that comprises or consist of the nucleotide sequence set forth in SEQ ID NO: 5

## Claims

1. One or more nucleotide sequences encoding one or more of the peptide sequences set forth in SEQ ID NO: AA1, SEQ ID NO: AA2, SEQ ID NO: AA3, and SEQ ID NO: AA4.

2. The one or more nucleotide sequences according to claim 1, wherein the nucleotide sequences are one or more of the sequences set forth in SEQ ID NO: 1 to SEQ ID NO:6.

3. A vector comprising one or more of the sequences set forth in SEQ ID NO: 1 to SEQ ID NO:6.

4. A cell or cell line comprising the vector according to claim 3.

5. The cell or cell line according to any of the preceding claims, wherein the cell or cell line comprises a first heterologous polynucleotide comprising a heterologous cis-acting regulatory sequence operably linked to a downstream promoter sequence, wherein said promoter is operably linked to an open reading frame encoding a luciferase reporter protein, wherein said open reading frame comprises or consist of the nucleotide sequence set forth in SEQ ID NO: 1 or SEQ ID NO: 2, and optionally comprising a further construct for constitutive production of a second reporter protein that is different from SEQ ID NO:AA1 and SEQ ID NO:AA2.

6. The cell or cell line according to any of the preceding claims, wherein the cell or cell line further comprises SEQ ID NO:3 and SEQ ID NO:4.

7. The cell or cell line according to any of the preceding claims, wherein the cell or cell line comprises a first heterologous polynucleotide comprising a heterologous cis-acting regulatory sequence operably linked to a downstream promoter sequence, wherein said promoter is operably linked to an open reading frame encoding a luciferase reporter protein, wherein said open reading frame comprises or consist of the nucleotide sequence set forth in SEQ ID NO: 1 or SEQ ID NO: 2, and optionally comprising a further construct comprising a heterologous cis-acting regulatory sequence operably linked to a downstream promoter sequence, wherein said promoter is operably linked to an open reading frame encoding a luciferase reporter protein such as firefly luciferase that requires a luciferin-based substrate, optionally comprising a further construct comprising a third heterologous cis-acting regulatory sequence operably linked to a downstream promoter sequence, wherein said promoter is operably linked to an open reading frame encoding a luciferase reporter protein that requires a colenterazine-based substrate that is different from SEQ ID NO:AA1 and SEQ ID NO:AA2 such as Renilla luciferase.

8. The cell or cell line according to any of the preceding claims, wherein the cell or cell line is a metazoan cell or cell line, selected from the group comprising HEK293, HEK293T, K562, U937, Jurkat, Molt-4, HeLa cells, HT1080 cells, ARPE-19, ARPE-19/HPV-16, insect cell such as Sf9 cells, avian cells such as DT-40, MSB1, or LMH, mouse cells such as L929 cells or the LS variant, Chinese hamster ovary (CHO) cells such as CHO-K1, CHO-DXB11, CHO-DG44, CHOK1SV cells including all variants.

9. Use of a cell or cell line according to any one of claims 4-8 or a sequence according to any one of claims 1-2 in a diagnostic method or drug screening method.

10. Use according to claim 9, wherein the diagnostic use may be e.g. a method for detecting and optionally quantitating the activity of a pharmacologically active molecule in a test sample, and wherein the drug screening method is a high throughput screening method.

11. A method for detecting and optionally quantitating the activity of a pharmacologically active molecule in a test sample, the method comprising the steps of:
i) providing a test sample,
ii) contacting said test sample with the cell line according to claim 5-7, that responds to treatment of the cell line with the pharmacology active molecule(s) present in the test sample, operably linked to a downstream promoter sequence, wherein said promoter is operably linked to an open reading frame encoding a first reporter protein comprising one of the sequences set forth in SEQ ID NO: AA1 and SEQ ID NO: AA2, and comprising a further construct for constitutive production of a second reporter protein that is different from SEQ ID NO:AA1 and SEQ ID NO:AA2,
iii) determining the activity of the first reporter protein as set forth in SEQ ID NO:AA1 or SEQ ID NO: AA2, using a colenterazine based substrate,
iv) determining the activity of the second reporter protein in said cell line after the reporter protein of SEQ ID NO:AA1 or SEQ ID NO: AA2 is measured in the same sample, by using a commercially available luciferin based substrate, that efficiently inhibits the activity of the first reporter protein comprising one the sequences set forth in SEQ ID NO: 1 and SEQ ID NO: 2 in the absence of colenterazine,
v) normalizing the activity of the first reporter protein activity relative to the activity of the second reporter protein activity.

12. A method for high throughput screening in drug discovery, the method comprising the steps of;
(i) providing a test sample(s), comprising a library of pharmacology active molecule(s) to be screened,
(ii) contacting said test sample(s) with the cell line according claim 5-7, said cell line comprising a first heterologous polynucleotide comprising a heterologous cis-acting regulatory sequence, that responds to treatment of the cell line with the pharmacology active molecule(s) present in the test sample(s), operably linked to a downstream promoter sequence, wherein said promoter is operably linked to an open reading frame encoding a first reporter protein comprising one the sequences set forth in SEQ ID NO: 1 and SEQ ID NO: 2.
(iii) Treating the cell line according to claims 5-7 with the agent(s) or drug candidates used to screen the library,
(iv) optionally, to provide for a normalization of the assay, the cell lines according to claim 5-7 further have a construct for the constitutive production of a luciferase that is different from that used in the reporter gene construct and thus different from the reporter proteins encoded by SEQ ID NO:1 or SEQ ID NO:2,
(v) determining the activity of the first reporter protein in said cell line using a coelenterazine based substrate or any other commercially available coelenterazine based substrate,
(vi) determining the activity of the second reporter protein in said cell line after the reporter gene luciferase is measured in the same sample using a commercially available luciferin based substrate that efficiently inhibits the activity of the first reporter protein comprising one the sequences set forth in SEQ ID NO: 1 and SEQ ID NO: 2 in the absence of coelenterazine, and
(vii) determining the activity of the first luciferase normalized relative to the activity of the second luciferase.

13. A method for detecting and optionally quantitating neutralizing antibodies against a pharmacology active molecule(s) present in the test sample, the method comprising the steps of;
(i) providing a test sample comprising a pharmacologically active molecule(s),
(ii) providing a first and a second cell sample, wherein said cells samples comprises the cell line according to claim 5-7,
(iii) contacting the first cell sample in (ii) with the with the pharmacology active molecule and subsequently test sample (in (i)),
(iv) contacting the second cell sample with the pharmacology active molecule,
(iv) determining the activity of the first reporter protein in the cells of the first cell sample and determining the activity of the first reporter protein in the cells of the second cell sample,
(v) assessing the ratio between the reporter activity in first and a second cell sample, where a ratio (first/second) lower than one is indicative for the presence of antibodies against the pharmacology active molecule in said sample.


SEQUENCE LISTING

<110>  SVAR Life Science AB

<120>  Novel Luciferases with improved properties

<130>  P82102338EP00

<160>  10

<170>  PatentIn version 3.5

<210>  1
<211>  450
<212>  DNA
<213>  Artificial

<220>
<223>  Artifical Sequence

<400>  1
atggaggccg aggccgagag aggcaagagc cccggcaaga agctgcccct ggccgtgatc      60

atggagatcg aggccaacgc cttcaaggcc ggctgcacca gaggctgcct gatctgcctg     120

agcaagatca agtgcaccgc caagatgaag gtgtacatcc ccggcagatg ccacgactac     180

ggcggcgaca agaagaccgg ccaggccggc atcgtgggcg ccatcgtgga catccccgag     240

atcagcggct tcaaggagat ggagcccatg gagcagttca tcgcccaggt ggacagatgc     300

gccagctgca ccaccggctg cctgaagggc ctggccaacg tgaagtgcag cgagctgctg     360

aagaagtggc tgcccgacag atgcgccagc ttcgccgaca agatccagaa ggaggtgcac     420

aacatcaagg gcatggccgg cgacagatga                                      450


<210>  2
<211>  450
<212>  DNA
<213>  Artificial

<220>
<223>  Artifical sequence

<400>  2
atggaggccg aggccgagag aggcaagagc cccggcaaga agctgcccct ggccgtgatc      60

atggagatcg aggccaacgc cttcaaggcc ggctgcacca gaggctgcct gatctgcctg     120

agcaagatca agtgcaccgc caagatgaag gtgtacatcc ccggcagatg ccacgactac     180

ggcggcgaca agaagaccgg ccaggccggc atcgtgggcg ccatcgtgga catccccgag     240

atcagcggct tcaaggagat ggagcccatg gagcagttcg tggcccaggt ggacagatgc     300

gccagctgca ccaccggctg cctgaagggc ctggccaacg tgaagtgcag cgagctgctg     360

aagaagtggc tgcccgacag atgcgccagc ttcgccgaca agatccagaa ggaggtgcac     420

aacatcaagg gcatggccgg cgacagatga                                      450

<210>  3
<211>  54
<212>  DNA
<213>  Artificial Sequence

<220>
<223>  Artifical sequence

<400>  3
atgggagtca aagttctgtt tgccctgatc tgcatcgctg tggccgaggc caag          54


<210>  4
<211>  573
<212>  DNA
<213>  Artificial Sequence

<220>
<223>  Artifical sequence

<400>  4
atggggacta ccctcgatga cacagcttac cgctatcgca cgtcagtgcc aggtgacgca      60

gaagccattg aggcgttgga tggttccttc acgaccgaca ccgtgtttcg cgttactgcg     120

acaggcgacg gtttcacctt acgcgaagtt ccggttgatc ctccgctgac caaagtgttt     180

ccggatgacg aaagcgacga cgaatcggat gatggggagg atggtgatcc cgatagccgc     240

acctttgtcg cctatggcga tgatggcgat ctggcaggct tcgttgtggt cagctactct     300

ggctggaatc gccgtcttac ggtggaggac atcgaagtag cgccggaaca tcgcggtcat     360

ggagtaggac gtgctctgat ggggttagcg acggagtttg ctcgggaacg tggtgcaggc     420

cacttgtggc tggaagtcac caacgtgaac gccccagcaa ttcacgccta tcgtcgtatg     480

ggctttacgc tgtgtggtct ggataccgcg ctgtatgacg gcactgcgag tgatggcgaa     540

caggccctgt acatgtcgat gccttgcccg taa                                  573


<210>  5
<211>  6325
<212>  DNA
<213>  Artificial Sequence

<220>
<223>  Artifical sequence

<400>  5
gagataccta cagcgtgagc tatgagaaag cgccacgctt cccgaaggga gaaaggcgga      60

caggtatccg gtaagcggca gggtcggaac aggagagcgc acgagggagc ttccaggggg     120

aaacgcctgg tatctttata gtcctgtcgg gtttcgccac ctctgacttg agcgtcgatt     180

tttgtgatgc tcgtcagggg ggcggagcct atggaaaaac gccagcaacg cggccttttt     240

acggttcctg gccttttgct ggccttttgc tcacatgttc tttcctgcgt tatcccctga     300

ttctgtggat aaccgtatta ccgcctttga gtgagctgat accgctcgcc gcagccgaac     360

gaccgagcgc agcgagtcag tgagcgagga agcggaagag cgcccaatac gcaaaccgcc 420

tctccccgcg cgttggccga ttcattaatg cagctggcac gacaggtttc ccgactggaa 480

agcgggcagt gagcgcaacg caattaatgt gagttagctc actcattagg caccccaggc 540

tttacacttt atgcttccgg ctcgtatgtt gtgtggaatt gtgagcggat aacaatttca 600

cacaggaaac agctatgacc atgattacgc caagcttaac cctagaaaga taatcatatt 660

gtgacgtacg ttaaagataa tcatgtgtaa aattgacgca tgtgttttat cggtctgtat 720

atcgaggttt atttattaat ttgaatagat attaagtttt attatattta cacttacata 780

ctaataataa attcaacaaa caatttattt atgtttattt atttattaaa aaaaacaaaa 840

actcaaaatt tcttctataa agtaacaaaa cttttatgag ggacagcccc cccccaaagc 900

ccccagggat gtaattacgt ccctcccccg ctagggggca gcagcgagcc gcccggggct 960

ccgctccggt ccggcgctcc ccccgcatcc ccgagccggc agcgtgcggg gacagcccgg 1020

gcacggggaa ggtggcacgg gatcgctttc ctctgaacgc ttctcgctgc tctttgagcc 1080

tgcagacacc tggggggata cggggaaaag gtacctgagc tcgctagcct cgagcctcca 1140

tagagagagg accatctcat ataaataggc catacccatg gagaaaggac attctaactg 1200

caacctttcg aagcctttgc tctggcacaa caggtagtag gcgacactgt tcgtgttgtc 1260

aacagatctg ccaccatggg agtcaaagtt ctgtttgccc tgatctgcat cgctgtggcc 1320

gaggccaaga tggaggccga ggccgagaga ggcaagagcc ccggcaagaa gctgcccctg 1380

gccgtgatca tggagatcga ggccaacgcc ttcaaggccg gctgcaccag aggctgcctg 1440

atctgcctga gcaagatcaa gtgcaccgcc aagatgaagg tgtacatccc cggcagatgc 1500

cacgactacg gcggcgacaa gaagaccggc caggccggca tcgtgggcgc catcgtggac 1560

atccccgaga tcagcggctt caaggagatg gagcccatgg agcagttcat cgcccaggtg 1620

gacagatgcg ccagctgcac caccggctgc ctgaagggcc tggccaacgt gaagtgcagc 1680

gagctgctga agaagtggct gcccgacaga tgcgccagct tcgccgacaa gatccagaag 1740

gaggtgcaca acatcaaggg catggccggc gacagatgat aattctagag tcggggcggc 1800

cggccgcttc gagcagacat gataagatac attgatgagt ttggacaaac cacaactaga 1860

atgcagtgaa aaaaatgctt tatttgtgaa atttgtgatg ctattgcttt atttgtaacc 1920

attataagct gcaataaaca agttaacaac aacaattgca ttcattttat gtttcaggtt 1980

cagggggagg tgtgggaggt tttttaaagc aagtaaaacc tctacaaatg tggtaaaatc 2040

gataaggatc cgtttgcgta ttgggcgctc ttccgctgat ctgcgcagca ccatggcctg 2100

aaataacctc tgaaagagga acttggttag ctaccttctg aggcggaaag aaccagctgt 2160

ggaatgtgtg tcagttaggg tgtggaaagt ccccaggctc cccagcaggc agaagtatgc 2220

aaagcatgca tctcaattag tcagcaacca ggtgtggaaa gtccccaggc tccccagcag 2280

gcagaagtat gcaaagcatg catctcaatt agtcagcaac catagtcccg cccctaactc 2340

cgcccatccc gcccctaact ccgcccagtt ccgcccattc tccgccccat ggctgactaa 2400

ttttttttat ttatgcagag gccgaggccg cctctgcctc tgagctattc cagaagtagt 2460

gaggaggctt ttttggaggc ctaggctttt gcaaaaagct cgattcttct gacactagca 2520

ctagtgccac catgaagaag cccgagctga ccgccaccag cgtggagaag ttcctgatcg 2580

agaagttcga cagcgtgagc gacctgatgc agctgagcga gggcgaggag agcagagcct 2640

tcagcttcga cgtgggcggc agaggctacg tgctgagagt gaacagctgc gccgacggct 2700

tctacaagga cagatacgtg tacagacact tcgccagcgc cgccctgccc atccccgagg 2760

tgctggacat cggcgagttc agcgagagcc tgacctactg catcagcaga agagcccagg 2820

gcgtgaccct gcaggacctg cccgagaccg agctgcccgc cgtgctgcag cccgtggccg 2880

aggccatgga cgccatcgcc gccgccgacc tgagccagac cagcggcttc ggccccttcg 2940

gcccccaggg catcggccag tacaccacct ggagagactt catctgcgcc atcgccgacc 3000

cccacgtgta ccactggcag accgtgatgg acgacaccgt gagcgccagc gtggcccagg 3060

ccctggacga gctgatgctg tgggccgagg actgccccga ggtgagacac ctggtgcacg 3120

ccgacttcgg cagcaacaac gtgctgaccg acaacggcag aatcaccgcc gtgatcgact 3180

ggagcgaggc catgttcggc gacagccagt acgaggtggc caacatcttc ttctggagac 3240

cctggctggc ctgcatggag cagcagacca gatacttcga gagaagacac cccgagctgg 3300

ccggcagccc cagactgaga gcctacatgc tgagaatcgg cctggaccag ctgtaccaga 3360

gcctggtgga cggcaacttc gacgacgccg cctgggccca gggcagatgc gacgccatcg 3420

tgagaagcgg cgccggcacc gtgggcagaa cccagatcgc cagaagaagc gccgccgtgt 3480

ggaccgacgg ctgcgtggag gtgctggccg acagcggcaa cagaagaccc agcaccagac 3540

ccagagccaa ggagtgaatg agtcgacaat caacctctgg attacaaaat ttgtgaaaga 3600

ttgactggta ttcttaacta tgttgctcct tttacgctat gtggatacgc tgctttaatg 3660

cctttgtatc atgcgttaac taaacttgtt tattgcagct tataatggtt acaaataaag 3720

caatagcatc acaaatttca caaataaagc atttttttca ctgcattcta gttgtggttt 3780

gtccaaactc atcaatgtat cttatcatgt ctggaattga ctcaaatgat gtcaattagt 3840

ctatcagaag ctcatctggt ctcccttccg ggggacaaga catccctgtt taatatttaa 3900

acagcagtgt tcccaaactg ggttcttata tcccttgctc tggtcaacca ggttgcaggg 3960

tttcctgtcc tcacaggaac gaagtcccta aagaaacagt ggcagccagg tttagccccg 4020

gaattgactg gattcctttt ttagggccca ttggtatggc tttttccccg tatcccccca 4080

ggtgtctgca ggctcaaaga gcagcgagaa gcgttcagag gaaagcgatc ccgtgccacc 4140

ttccccgtgc ccgggctgtc cccgcacgct gccggctcgg ggatgcgggg ggagcgccgg 4200

accggagcgg agccccgggc ggctcgctgc tgccccctag cgggggaggg acgtaattac 4260

atccctgggg gctttggggg ggggctgtcc ctgatatcta taacaagaaa atatatatat 4320

aataagttat cacgtaagta gaacatgaaa taacaatata attatcgtat gagttaaatc 4380

ttaaaagtca cgtaaaagat aatcatgcgt cattttgact cacgcggtcg ttatagttca 4440

aaatcagtga cacttaccgc attgacaagc acgcctcacg ggagctccaa gcggcgactg 4500

agatgtccta aatgcacagc gacggattcg cgctatttag aaagagagag caatatttca 4560

agaatgcatg cgtcaatttt acgcagacta tctttctagg gttaagaatt cactggccgt 4620

cgttttacaa cgtcgtgact gggaaaaccc tggcgttacc caacttaatc gccttgcagc 4680

acatccccct ttcgccagct ggcgtaatag cgaagaggcc cgcaccgatc gcccttccca 4740

acagttgcgc agcctgaatg gcgaatggcg cctgatgcgg tattttctcc ttacgcatct 4800

gtgcggtatt tcacaccgca tatggtgcac tctcagtaca atctgctctg atgccgcata 4860

gttaagccag ccccgacacc cgccaacacc cgctgacgcg ccctgacggg cttgtctgct 4920

cccggcatcc gcttacagac aagctgtgac cgtctccggg agctgcatgt gtcagaggtt 4980

ttcaccgtca tcaccgaaac gcgcgagacg aaagggcctc gtgatacgcc tatttttata 5040

ggttaatgtc atgataataa tggtttctta gttgacaatt aatcatcggc atagtatatc 5100

ggcatagtat aatacgactc actataggag ggccatcatg gggactaccc tcgatgacac 5160

agcttaccgc tatcgcacgt cagtgccagg tgacgcagaa gccattgagg cgttggatgg 5220

ttccttcacg accgacaccg tgtttcgcgt tactgcgaca ggcgacggtt tcaccttacg 5280

cgaagttccg gttgatcctc cgctgaccaa agtgtttccg gatgacgaaa gcgacgacga 5340

atcggatgat ggggaggatg gtgatcccga tagccgcacc tttgtcgcct atggcgatga 5400

tggcgatctg gcaggcttcg ttgtggtcag ctactctggc tggaatcgcc gtcttacggt 5460

ggaggacatc gaagtagcgc cggaacatcg cggtcatgga gtaggacgtg ctctgatggg 5520

gttagcgacg gagtttgctc gggaacgtgg tgcaggccac ttgtggctgg aagtcaccaa 5580

cgtgaacgcc ccagcaattc acgcctatcg tcgtatgggc tttacgctgt gtggtctgga 5640

taccgcgctg tatgacggca ctgcgagtga tggcgaacag gccctgtaca tgtcgatgcc 5700

ttgcccgtaa gagtcaggca actatggatg aacgaaatag acagatcgct gagataggtg 5760

cctcactgat taagcattgg taactgtcag accaagttta ctcatatata ctttagattg 5820

atttaaaact tcatttttaa tttaaaagga tctaggtgaa gatccttttt gataatctca 5880

tgaccaaaat cccttaacgt gagttttcgt tccactgagc gtcagacccc gtagaaaaga 5940

tcaaaggatc ttcttgagat cctttttttc tgcgcgtaat ctgctgcttg caaacaaaaa 6000

aaccaccgct accagcggtg gtttgtttgc cggatcaaga gctaccaact ctttttccga 6060

aggtaactgg cttcagcaga gcgcagatac caaatactgt tcttctagtg tagccgtagt 6120

taggccacca cttcaagaac tctgtagcac cgcctacata cctcgctctg ctaatcctgt 6180

taccagtggc tgctgccagt ggcgataagt cgtgtcttac cgggttggac tcaagacgat 6240

agttaccgga taaggcgcag cggtcgggct gaacgggggg ttcgtgcaca cagcccagct 6300

tggagcgaac gacctacacc gaact 6325

<210> 6
<211> 2235
<212> DNA
<213> Artificial Sequence

<220>
<223> Artifical sequence

<400> 6
atgggagtca aagttctgtt tgccctgatc tgcatcgctg tggccgaggc caagatggag 60

gccgaggccg agagaggcaa gagccccggc aagaagctgc ccctggccgt gatcatggag 120

atcgaggcca acgccttcaa ggccggctgc accagaggct gcctgatctg cctgagcaag 180

atcaagtgca ccgccaagat gaaggtgtac atccccggca gatgccacga ctacggcggc 240

gacaagaaga ccggccaggc cggcatcgtg ggcgccatcg tggacatccc cgagatcagc 300

ggcttcaagg agatggagcc catggagcag ttcatcgccc aggtggacag atgcgccagc 360

tgcaccaccg gctgcctgaa gggcctggcc aacgtgaagt gcagcgagct gctgaagaag 420

tggctgcccg acagatgcgc cagcttcgcc gacaagatcc agaaggaggt gcacaacatc 480

aagggcatgg ccggcgacag aaaaattgtc gctcctgtca aacaaactct taactttgat 540

ttactcaaac tggctgggga tgtagaaagc aatccaggtc caatggagga cgcaaagaac 600

atcaagaagg gtcctgcacc cttttaccct ctggaggacg gaactgctgg cgagcaactg 660

cacaaagcca tgaaacgtta tgctctggta cctgggacta tagcctttac ggatgcacac 720

attgaggtgg acatcacgta tgccgagtat ttcgaaatga gtgttcgact ggcagaagcg 780

atgaagaggt acgggttgaa cacaaatcac aggatagtgg tctgctcaga gaactccctg 840

cagttcttca tgcctgtcct cggcgcactg tttatcgggg tcgcggttgc tcccgctaat 900

gacatctaca acgaacggga actgctgaac tccatgggca taagccagcc gacagtcgtg 960

ttcgtgtcca agaaagggct ccagaaaatt ctgaacgtcc agaagaagct gcccattatc 1020

cagaaaatca tcatcatgga cagcaaaacc gactatcaag ggttccagtc catgtacacg 1080

ttcgtgactt cccatctgcc accaggcttc aatgagtacg attttgtccc agagtcattt 1140

gacaggacaa aaaccattgc cttgatcatg aactcttccg gttctacagg tctgccaaag 1200

ggagtggcat tgccccatcg tactgcctgt gtgcgattta gccacgctag ggaccccata 1260

tttggtaatc agattatccc agatactgca atcctgtcag tggtgccatt tcatcacggt 1320

ttcggcatgt tcaccaccct tggctacctc atctgcggat ttagagtggt cctcatgtac 1380

cggttcgaag aggagttgtt cctgcgctct cttcaggatt acaaaattca gagcgctctc 1440

ctggtgccta cactctttag cttcttcgcc aagtctacac tcattgacaa atatgacctg 1500

agtaatctcc acgagattgc ctctggcggg gcacctctga gtaaggaggt gggagaagcc 1560

gtggccaagc ggtttcacct cccaggaata cgccaagggt atggtctgac cgaaaccacc 1620

tcagccatct tgatcacacc agaaggggac gataaacccg gagcggtggg caaggttgtc 1680

cctttctttg aggcaaaggt agttgacctg gatactggca aaacccttgg cgtgaatcag 1740

agaggcgaac tgtgtgtgag aggacccatg attatgagcg ggtacgtcaa caatcccgag 1800

gccaccaatg cgctgatcga caaggatgga tggctgcaca gtggcgatat tgcctattgg 1860

gatgaggatg agcatttctt cattgtggac cgcctgaaaa gcctcataaa gtacaagggg 1920

taccaggttg ctccggccga acttgagagc atactgctgc aacatccgaa catctttgat 1980

gctggtgttg ccggccttcc cgatgatgat gctggagagc tgcctgcagc tgttgtggta 2040

ctggaacatg gcaaaaccat gacagagaaa gaaattgtcg actatgtggc ctctcaggtt 2100

accacagcca agaagttgcg gggaggagta gtctttgtgg acgaagtacc caaaggcctt 2160

actgggaagc ttgatgccag aaagatccgc gaaattctga tcaaagcgaa gaagggcggt 2220

aaaattgctg tgtag 2235

<210> 7
<211> 161
<212> PRT
<213> Artificial Sequence

<220>
<223> Artifical sequence

<400> 7

Met Gly Val Lys Val Leu Phe Ala Leu Ile Cys Ile Ala Val Ala Glu
1               5                   10                  15

Ala Lys Arg Gly Lys Ser Pro Gly Lys Lys Leu Pro Leu Ala Val Ile
            20                  25                  30

Met Glu Ile Glu Ala Asn Ala Phe Lys Ala Gly Cys Thr Arg Gly Cys
        35                  40                  45

Leu Ile Cys Leu Ser Lys Ile Lys Cys Thr Ala Lys Met Lys Val Tyr
    50                  55                  60

Ile Pro Gly Arg Cys His Asp Tyr Gly Gly Asp Lys Lys Thr Gly Gln

65                  70                  75                  80

Ala Gly Ile Val Gly Ala Ile Val Asp Ile Pro Glu Ile Ser Gly Phe
                85                  90                  95

Lys Glu Met Glu Pro Met Glu Gln Phe Ile Ala Gln Val Asp Arg Cys
            100                 105                 110

Ala Ser Cys Thr Thr Gly Cys Leu Lys Gly Leu Ala Asn Val Lys Cys
        115                 120                 125

Ser Glu Leu Leu Lys Lys Trp Leu Pro Asp Arg Cys Ala Ser Phe Ala
    130                 135                 140

Asp Lys Ile Gln Lys Glu Val His Asn Ile Lys Gly Met Ala Gly Asp
145                 150                 155                 160

Arg

<210>  8
<211>  161
<212>  PRT
<213>  Artificial Sequence

<220>
<223>  Artificial sequence

<400>  8

Met Gly Val Lys Val Leu Phe Ala Leu Ile Cys Ile Ala Val Ala Glu
1               5                   10                  15

Ala Lys Arg Gly Lys Ser Pro Gly Lys Lys Leu Pro Leu Ala Val Ile
            20                  25                  30

Met Glu Ile Glu Ala Asn Ala Phe Lys Ala Gly Cys Thr Arg Gly Cys
        35                  40                  45

Leu Ile Cys Leu Ser Lys Ile Lys Cys Thr Ala Lys Met Lys Val Tyr
    50                  55                  60

Ile Pro Gly Arg Cys His Asp Tyr Gly Gly Asp Lys Lys Thr Gly Gln
65                  70                  75                  80

Ala Gly Ile Val Gly Ala Ile Val Asp Ile Pro Glu Ile Ser Gly Phe
                85                  90                  95

Lys Glu Met Glu Pro Met Glu Gln Phe Val Ala Gln Val Asp Arg Cys
            100                 105                 110

Ala Ser Cys Thr Thr Gly Cys Leu Lys Gly Leu Ala Asn Val Lys Cys
        115                 120                 125

Ser Glu Leu Leu Lys Lys Trp Leu Pro Asp Arg Cys Ala Ser Phe Ala
    130                 135                 140

Asp Lys Ile Gln Lys Glu Val His Asn Ile Lys Gly Met Ala Gly Asp
145                 150                 155                 160

Arg

<210>  9
<211>  18
<212>  PRT
<213>  Artificial Sequence

<220>
<223>  Artificial sequence

<400>  9

Met Gly Val Lys Val Leu Phe Ala Leu Ile Cys Ile Ala Val Ala Glu
1               5                   10                  15

Ala Lys

<210>  10
<211>  726
<212>  PRT
<213>  Artificial Sequence

<220>
<223>  Artifical sequence

<400>  10
   Met Glu Ala Glu Ala Glu Arg Gly Lys Ser Pro Gly Lys Lys Leu Pro
   1               5                   10                  15
   Leu Ala Val Ile Met Glu Ile Glu Ala Asn Ala Phe Lys Ala Gly Cys
               20                  25                  30
   Thr Arg Gly Cys Leu Ile Cys Leu Ser Lys Ile Lys Cys Thr Ala Lys
           35                  40                  45
   Met Lys Val Tyr Ile Pro Gly Arg Cys His Asp Tyr Gly Gly Asp Lys
       50                  55                  60
   Lys Thr Gly Gln Ala Gly Ile Val Gly Ala Ile Val Asp Ile Pro Glu
   65                  70                  75                  80
   Ile Ser Gly Phe Lys Glu Met Glu Pro Met Glu Gln Phe Ile Ala Gln
                   85                  90                  95
   Val Asp Arg Cys Ala Ser Cys Thr Thr Gly Cys Leu Lys Gly Leu Ala
               100                 105                 110
   Asn Val Lys Cys Ser Glu Leu Leu Lys Lys Trp Leu Pro Asp Arg Cys
           115                 120                 125
   Ala Ser Phe Ala Asp Lys Ile Gln Lys Glu Val His Asn Ile Lys Gly
       130                 135                 140
   Met Ala Gly Asp Arg Lys Ile Val Ala Pro Val Lys Gln Thr Leu Asn
   145                 150                 155                 160
   Phe Asp Leu Leu Lys Leu Ala Gly Asp Val Glu Ser Asn Pro Gly Pro
                   165                 170                 175
   Met Glu Asp Ala Lys Asn Ile Lys Lys Gly Pro Ala Pro Phe Tyr Pro
               180                 185                 190
   Leu Glu Asp Gly Thr Ala Gly Glu Gln Leu His Lys Ala Met Lys Arg
           195                 200                 205
   Tyr Ala Leu Val Pro Gly Thr Ile Ala Phe Thr Asp Ala His Ile Glu
       210                 215                 220
   Val Asp Ile Thr Tyr Ala Glu Tyr Phe Glu Met Ser Val Arg Leu Ala
   225                 230                 235                 240
   Glu Ala Met Lys Arg Tyr Gly Leu Asn Thr Asn His Arg Ile Val Val
                   245                 250                 255
   Cys Ser Glu Asn Ser Leu Gln Phe Phe Met Pro Val Leu Gly Ala Leu
               260                 265                 270
   Phe Ile Gly Val Ala Val Ala Pro Ala Asn Asp Ile Tyr Asn Glu Arg
           275                 280                 285
   Glu Leu Leu Asn Ser Met Gly Ile Ser Gln Pro Thr Val Val Phe Val
       290                 295                 300
   Ser Lys Lys Gly Leu Gln Lys Ile Leu Asn Val Gln Lys Lys Leu Pro
   305                 310                 315                 320
   Ile Ile Gln Lys Ile Ile Ile Met Asp Ser Lys Thr Asp Tyr Gln Gly
                   325                 330                 335
   Phe Gln Ser Met Tyr Thr Phe Val Thr Ser His Leu Pro Pro Gly Phe
               340                 345                 350
   Asn Glu Tyr Asp Phe Val Pro Glu Ser Phe Asp Arg Asp Lys Thr Ile
           355                 360                 365
   Ala Leu Ile Met Asn Ser Ser Gly Ser Thr Gly Leu Pro Lys Gly Val
       370                 375                 380
   Ala Leu Pro His Arg Thr Ala Cys Val Arg Phe Ser His Ala Arg Asp
   385                 390                 395                 400
   Pro Ile Phe Gly Asn Gln Ile Ile Pro Asp Thr Ala Ile Leu Ser Val
                   405                 410                 415
   Val Pro Phe His His Gly Phe Gly Met Phe Thr Thr Leu Gly Tyr Leu
               420                 425                 430
   Ile Cys Gly Phe Arg Val Val Leu Met Tyr Arg Phe Glu Glu Glu Leu
           435                 440                 445
   Phe Leu Arg Ser Leu Gln Asp Tyr Lys Ile Gln Ser Ala Leu Leu Val
       450                 455                 460
   Pro Thr Leu Phe Ser Phe Phe Ala Lys Ser Thr Leu Ile Asp Lys Tyr
   465                 470                 475                 480
   Asp Leu Ser Asn Leu His Glu Ile Ala Ser Gly Gly Ala Pro Leu Ser
                   485                 490                 495
   Lys Glu Val Gly Glu Ala Val Ala Lys Arg Phe His Leu Pro Gly Ile
               500                 505                 510
   Arg Gln Gly Tyr Gly Leu Thr Glu Thr Thr Ser Ala Ile Leu Ile Thr
           515                 520                 525
   Pro Glu Gly Asp Asp Lys Pro Gly Ala Val Gly Lys Val Val Pro Phe
       530                 535                 540
   Phe Glu Ala Lys Val Val Asp Leu Asp Thr Gly Lys Thr Leu Gly Val
   545                 550                 555                 560
   Asn Gln Arg Gly Glu Leu Cys Val Arg Gly Pro Met Ile Met Ser Gly
                   565                 570                 575
   Tyr Val Asn Asn Pro Glu Ala Thr Asn Ala Leu Ile Asp Lys Asp Gly
               580                     585                     590
   Trp Leu His Ser Gly Asp Ile Ala Tyr Trp Asp Glu Asp Glu His Phe
           595                     600                     605
   Phe Ile Val Asp Arg Leu Lys Ser Leu Ile Lys Tyr Lys Gly Tyr Gln
       610                     615                     620
   Val Ala Pro Ala Glu Leu Glu Ser Ile Leu Leu Gln His Pro Asn Ile
   625                     630                     635                     640
   Phe Asp Ala Gly Val Ala Gly Leu Pro Asp Asp Asp Ala Gly Glu Leu
                   645                     650                     655
   Pro Ala Ala Val Val Val Leu Glu His Gly Lys Thr Met Thr Glu Lys
               660                     665                     670
   Glu Ile Val Asp Tyr Val Ala Ser Gln Val Thr Thr Ala Lys Lys Leu
           675                     680                     685
   Arg Gly Gly Val Val Phe Val Asp Glu Val Pro Lys Gly Leu Thr Gly
       690                     695                     700
   Lys Leu Asp Ala Arg Lys Ile Arg Glu Ile Leu Ile Lys Ala Lys Lys
   705                     710                     715                     720
   Gly Gly Lys Ile Ala Val
                   725